(19) Europäisches Patentamt
European Patent Office
Office européen des brevets

(11) Publication number : **0 484 265 A1**

(12) **EUROPEAN PATENT APPLICATION**

(21) Application number : **91500118.4**

(22) Date of filing : **24.10.91**

(51) Int. Cl.$^5$ : **C07D 401/12**, A61K 31/415, A61K 31/44

(30) Priority : **31.10.90 ES 9002764**

(43) Date of publication of application : **06.05.92 Bulletin 92/19**

(84) Designated Contracting States : **DE ES FR GB IT**

(71) Applicant : **CENTRO GENESIS PARA LA INVESTIGACION, S.L.**
**Beethoven, 15, Sobreático 3a**
**E-08021 Barcelona (ES)**

(71) Applicant : **ESTEVE QUIMICA, S.A.**
**Avenida Mare de Deu de Montserrat, 12**
**E-08024 Barcelona (ES)**

(72) Inventor : **Palomo Coll, Alberto**
**Dr. Carulla, 10**
**E-08017 Barcelona (ES)**

(74) Representative : **Curell Sunol, Jorge et al**
**c/o Dr. Ing. M. Curell Sunol I.I. S.L. Passeig de Gràcia 65 bis**
**E-08008 Barcelona (ES)**

(54) **A process for the preparation of omeprazol.**

(57) The process starts by reacting 2,3,5 trimethylpyridine with hydrogen peroxide in the presence of catalysts, giving new reactive ionic species allowing the number of required steps to be substantially reduced. In the final important step, oxidation to omeprazol, there are used new salts of 5-methoxy-2-((3,5-dimethyl-4-methoxy-2-pyridine)methylthio)-1H-benzimidazole which, as the oxidation evolves, precipitate the omeprazol. The new oxidation method avoids superoxidations, provide for faster oxidation, high purity and yields of over 90%.

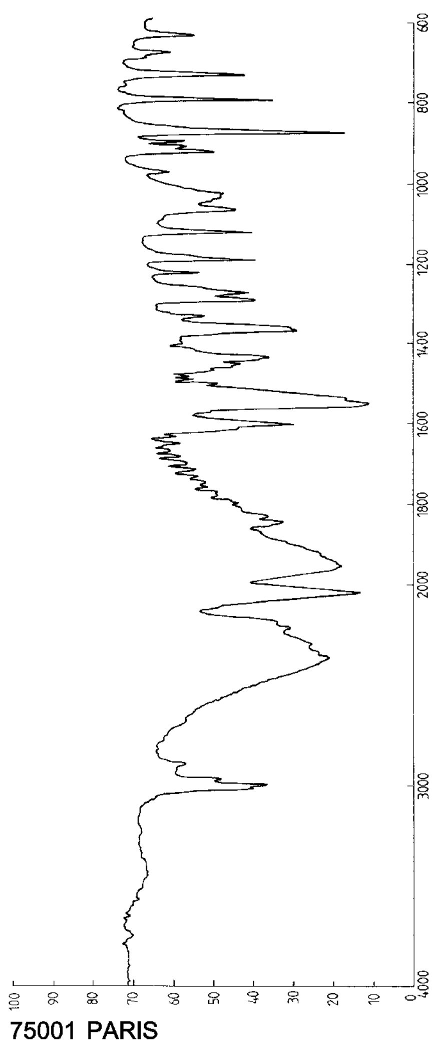


FIG. 1

EP 0 484 265 A1

The invention relates to a process for the preparation of omeprazol, the chemical name of which is 5-methoxy-2-(((3,5-dimethyl-4-methoxy-2-pyridinyl)methyl)sulfinyl)-1H-benzimidazole, of formula I

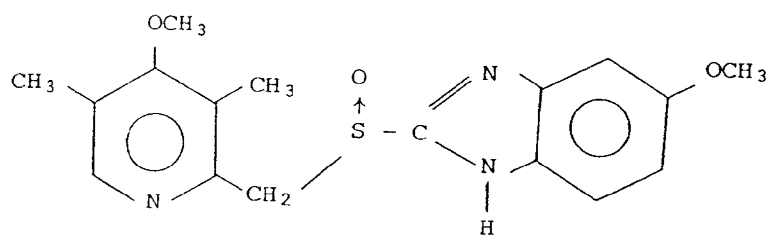

(I)

Omeprazol is a very effective drug for the treatment of gastric ulcer.

The only synthesis route for omeprazol described in the literature (Spanish patents nos. 525,122 and 527,171 and European patent no. 0 103 553) comprises 8 chemical steps corresponding to the following reactions carried out in the specified order: a) N-oxidation of 2,3,5-trimethylpyridine; b) nitration of the 4-position; c) nucleophilic substitution of the $-NO_2$ radical by the $-OCH_3$ radical; d) O-acylation and subsequent acetoxylation of the 2-position methyl group; e) base hydrolisis of the acetoxyl group; f) nucleophilic substitution of the -OH radical by the -Cl radical; g) nucleophilic substitution of the -Cl radical by the -SR radical, where R is

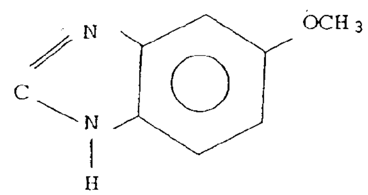

and finally, h) oxidation to omeprazol.

The step a) oxidation is carried out with $H_2O_2$ in acetic acid and requires some considerably long reaction times, about 30 hours. The oxidizing agent which really acts is the peracetic acid (a potentially explosive compound) formed in situ since the hydrogen peroxide alone is incapable of forming N-oxides. The 2,3,5-trimethylpyridine N-oxide is isolated from the reaction medium by extraction with an organic solvent and has the formula CI.

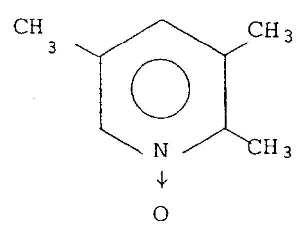

CI

The yield of this reaction is high, 96.6% (Spanish patent 525.122 and European patent 0 103 553).

The step b) nitration is carried out using $HNO_3$ in the presence of $H_2SO_4$ , under which conditions the nitrating agent has the formula $NO_2^+$ $HSO_4^-$. The 2,3,5-trimethyl-4-nitropyridine n-oxide thus formed, having formula CII

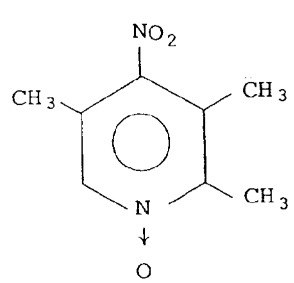

CII

is isolated by neutralization and extraction from the reaction medium using an organic solvent.

The nucleophilic substitution of step c) is carried out by generating sodium methoxide in situ by addition of NaOH over $CH_3OH$. The nucleophile of this reaction is the solvated anion $CH_3O^-$. $(CH_3OH)_x$ $(H_2O)_i$. The 2,3,5-trimethyl-4-methoxipyridine N-oxide of formula CIII

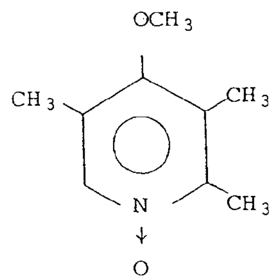

CIII

is obtained, the joint reaction yield of steps b) and c) being 72.5% (Spanish patent 525.122 and European patent 0 103 553). The claims of these two patents protect the structures CI, CII and CIII.

The literature offers an omeprazol synthesis design starting out from the compound of formula CIII, i.e., a sequence of intermediate structures, but does not provide any information on the physical and chemical reaction conditions of two steps, f) and g).

Starting out from the data provided by the literature, the present inventor has studied the possible line of preparation of omeprazol from compound CIII. Hereafter there are described the operative steps involved in this transformation; these operative steps include chemical reactions and physical operations of isolation of the various products.

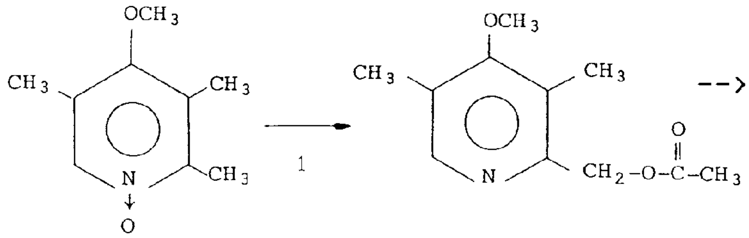

--> Distillation -->
2

alkaline
------>
hydrolysis

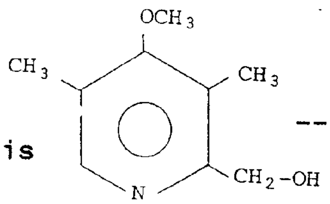

--> Extraction -->
4

Distillation -->
$CH_2Cl_2$
5

--> Product Distillation
6
(high resin content)

-->
7

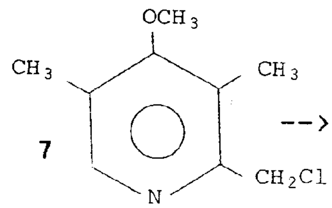

--> Distillation -->
8

Centrifugation
9

--> Drying -->
10

11

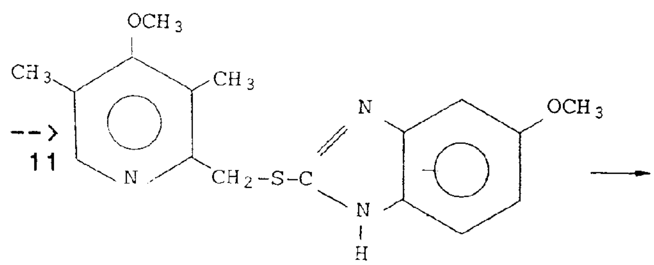

-->Distillation
12

--> Extraction
13

-->Omeprazol (in solution)
14

--> Vacuum Distillation
15

--> Preparation of impure Omeprazol, violet, in solid state
16

--> Centrifugation
17

As may be seen from the scheme, the number of operative steps required for the conversion of the compound CIII in omeprazol following the design described (Spanish patent 525.122 and European patent 0 103 553) is considerably high. Furthermore, the results obtained in several of them (yield, purity) are unsatisfactory.

The step d) acylation is carried out by treatment of the compound CIII with acetic anhydride in acetic acid and the step e) hydrolysis is carried out with aqueous NaOH under reflux. The alcohol obtained must be purified (distillation) prior to use in the step f). The joint yield of the steps d) and e), carried out under the above conditions, is scarcely 40%.

The step h) oxidation is carried out with metachloroperbenzoic acid in solution with an organochlorinated solvent at a temperature of 0 to -5°C, the yield described being 70% (Spanish patent of introduction 527.171).

An examination of this oxidation method has evidenced that the stepwise operation affords serious difficulties, since omeprazol is obtained in $CH\ Cl_3$ or $CH_2CL_2$ solution and the isolation thereof, on an industrial scale, requires a period of 5 hours distillation. The decomposition of the product because of the extended thermal effect and by the presence of cathalytic amounts of acids may be considered to be 60-75%.

Thus, a sample of white omeprazol dissolved in $CH_2CL_2$ (reagent quality) instantaneously develops at room temperature an intense violet colour which turns black with time, similar to what is observed when carboxylic acids are added.

The above drawbacks and those referred to are overcome by means of the process of the invention which is characterized by comprising the following stages: a′) oxidation of 2,3,5-trimethylpyridine by an oxidizing agent formed by the reaction product of hydrogen peroxide and a catalyst, said oxidation taking place in the presence of catalytic traces of an acid selected from the group formed by the alkylsulfonic and arylsulfonic acids and sulfuric acid, to give N-oxide derivatives; b′) reduction of the excess hydrogen peroxide to water by way of reducing agents; c′) fixation of the N-oxide with an acid; d′) distillation of the water until a low moisture content is obtained; e′) addition of a dehydrating agent to destroy the said low moisture content; f′) nitration by a nitronium salt of formula $A^-NO_2^+$, $A^-$ being acetate, a halogenide, a sulfonate or an oxyhalogenide of phosphorus oxidized in 3- or 5- position, to give a salt of a nitrated compound; and g′) distillation of the excess nitric acid; and in that, optionally after an halogenation step giving a compound of formula III

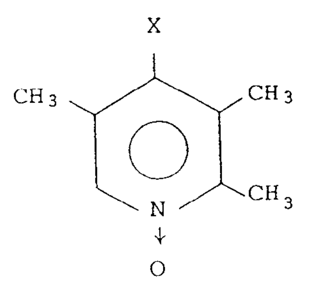

(III)

where X is a halogen, the following takes place:

either steps: 1) allyl chlorination of the 2-position methyl group; 2) nucleophilic substitution of the -Cl radical of the 2-position methyl group by the -SR radical, where R has the meaning given above; 3) deoxygenation of the N-oxide; 4) nucleophilic substitution of the 4-position substituent by the $-OCH_3$ radical; and 5) oxidation providing omeprazol, such that steps 1) to 4) may take place in any order provided that: step 2) is subsequent to step 1); and step 4) is either subsequent to step 2) or prior to step 1);

or, steps: 1′) O-acylation and subsequent acetoxylation of the 2-position methyl group; 2′) acid hydrolysis; 3′) nucleophilic substitution of the -OH radical by the -Cl radical; 4′) nucleophilic substitution of the -Cl radical of the 2-position methyl group by the -SR radical, where R has the meaning given above; 5′) nucleophilic substitution of the 4-position substituent by the $-OCH_3$ radical, after which an oxidation step providing the omeprazol is carried out.

This process leads to new structures in the literature which are characterized by their melting point and the ′H-NMR or IR spectra.

Before mentioning the structures, reference is made to drawings accompanying the present description.

In Figures 1 to 14 there are shown infrarred spectra of the compounds or new structure cited hereinafter. These spectra are represented in Cartesian graphs, where the X-axis gives the wave number expressed in $cm^{-1}$ and the Y-axis gives the transmission percentage. These spectra show the high purity of the products obtained by the process of the invention.

Figures 15 and 16 show ′H-NMR spectra and in them the X-axes represent the magnetic field force in parts per million (ppm).

The new compounds to which reference has been made are those given on the following pages:

i)

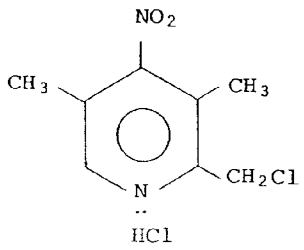

Melting point: 124-127 °C
IR: Fig. 1

ii)

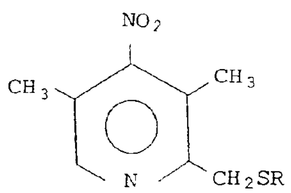

Melting point: 80-90 °C
IR: Fig. 2

iii)

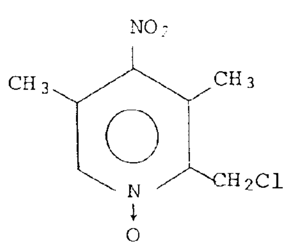

Melting point: 80-107 °C
IR: Fig. 3
'H-NMR: Fig. 15

iv)

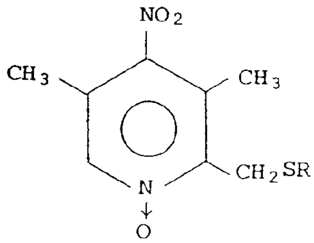

Melting point: 65-70 °C
IR: Fig. 4

v)

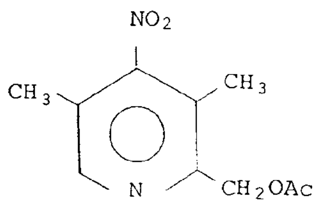

IR: Fig. 5

vi)

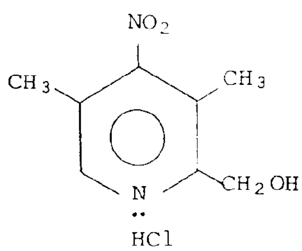

Melting point: 132-135 °C
IR: Fig. 6

vii)

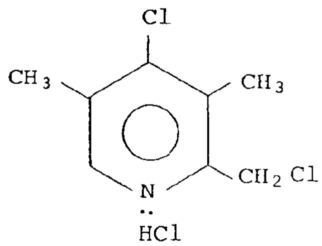

Melting point: 150-158 °C
IR: Fig. 7

viii)

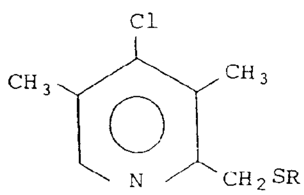

Melting point: 79-88 °C
IR: Fig. 8

ix)

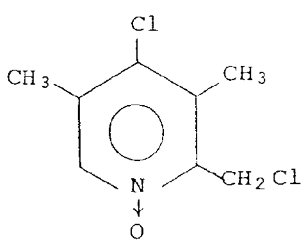

Melting point: 134-149 °C
IR: Fig. 9
'H-NMR: Fig. 16

x)

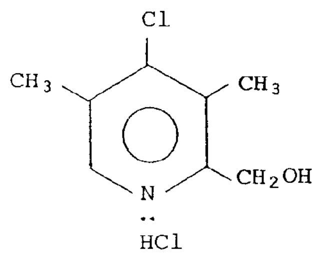

Melting point: 190-196 °C
IR: Fig. 10

xi)

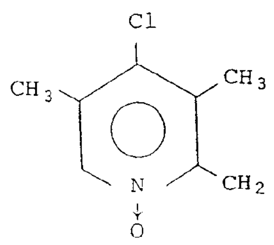

Melting point: 148-152 °C
IR: Fig. 11

xii)

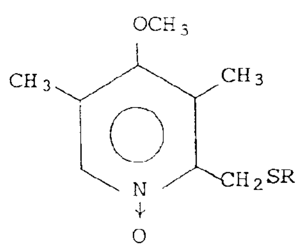

IR: Fig. 12

xiii)

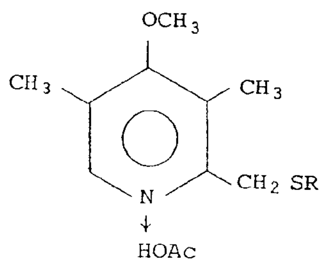

Melting point: 132-141 °C
IR: Fig. 13

xiv)

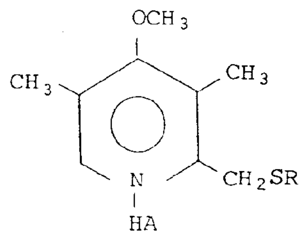

Melting point: 95-125 °C
IR: Fig. 14

A is 2-ethylhexanoate

The stage a') oxidation is carried out preferably at a temperature ranging from 30 to 100 °C, and the catalyst for said stage a) is selected from the group formed by: phosphotungstic acid, having the formula $H_3 (P(W_3O_{10})_4)$. .$xH_2O$; ammonium molybdate having the formula $(NH_4)_2MoO_4$; sodium tungstate having the formula $Na_2WO_4$ phosphomolybdic acid having the formula $H_3 (P(Mo_3O_{10})_4.xH_2O$; and silicotungstic acid having the formula $H_4 (Si(W_3O_{10})_4).xH_2O$. The oxidizing species of this reaction is the corresponding percatalyst (reaction product of the hydrogen peroxide with the catalyst used), which is non-explosive. The use of these catalysts, discovered by the inventor hereof, Alberto Palomo Coll, for the oxidation of various compounds other than those considered here, was described by the inventor in the following documents: Afinidad, 1988; 418; Afinidad, 1985, 397; Afinidad, 1986, 406 and Spanish patent 509.855 (1982).

Since 2,3,5-trimethylpyridine is highly insoluble in $H_2O_2$, the said stage a') oxidation is effective in the presence of a strong acid, which protonates the pyridine nitrogen with preference to the oxygen of the N-oxide formed, such that said acid acts as a "phase transfer catalyst".

If the stage d') distillation is carried out directly after stage a'), there is produced a steam entrainment of the N-oxide formed and a reversible chemical reaction: reduction of the N-oxide to the starting pyridine derivative due to the temperature and to the reducing properties of the $H_2O_2$. In a word, the product yield and purity are reduced. Furthermore, these drawbacks mean that the system would be aggravated on an industrial scale since, on increasing the distillation time, more reaction would take place.

These problems were solved by carrying out stages b') and c') prior to the distillation.

The reducing agent of stage b') is preferably hydrazine hydrate, sulfurous anhydride, a sulfite, a bisulfite or a metabisulfite, while the acid of stage c') is a strong organic acid, nitric acid or sulfuric acid.

In turn, the stage e') dehydrating agent is preferably acetic anhydride, phosphorus oxychloride, phosphorus pentachloride or phosphorus trichloride.

According to the invention, the stage f') nitronium salt is formed in situ by addition over nitric acid of a compound selected from the group formed by a sulfonic acid (preferably trifluoromethanesulfonic acid or Nafion-H), acetic anhydride and a phosphorus halogenide oxidized in 3- or 5- position, preferably phosphorus oxychloride, phosphorus trichloride or phosphorus pentachloride. These nitronium salts have not been described for the nitration of pyridine derivatives.

Also according to the invention, the pyridine ring halogenation step is carried out by direct treatment of said salt of a nitrated compound, previously neutralized with sodium acetate and acetic acid, with $X-CO-CH_3$ , $SOX_2$/ di-methylformamide, where X is halogen, at a temperature ranging from 0 to 55 °C, to give a compound of formula III.

Mention has been made above to steps which lead in succession to omeprazol, either starting out from said salt of a nitrated compound, or from the formula III compound; it was also said that these steps may be performed in any order provided they fulfil certain conditions.

Hereinafter there are described the preferred alternatives in the order of these steps:

A:

a) allyl chlorination of the 2-position methyl group, after neutralization, of said salt of a nitrated compound, by the addition of a halogenating agent, to give a compound of formula IV

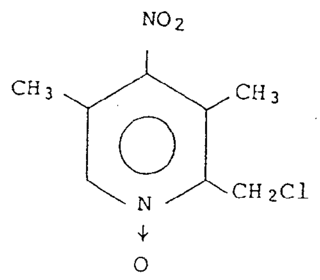

(IV)

b) nucleophilic substitution of the -Cl radical by the -SR radical, where R has the meaning given above, by reacting the compound of formula IV with 5-methoxy-2-mercapto-benzimidazole of formula V

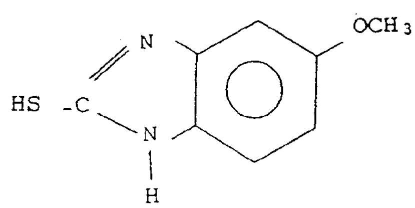

(V)

to give 5-methoxy-2-((3,5-dimethyl-4-nitro-2-pyridinyl-N-oxide)methylthio)-1H-benzimidazole of formula VI

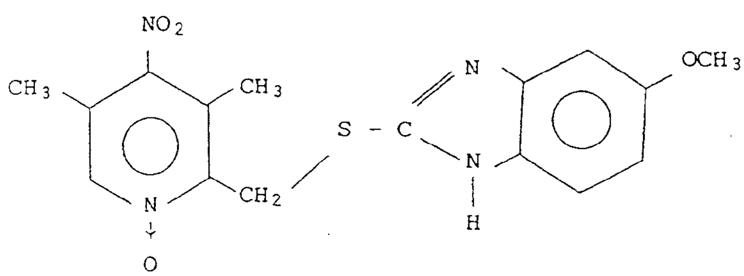

(VI)

c) deoxygenation of the formula VI compound by reaction with a reducing compound to give 5-methoxy-2-((3,5-dimethyl-4-nitro-2-pyridinyl)methyllhio)-1H-benzimidazole of formula VII

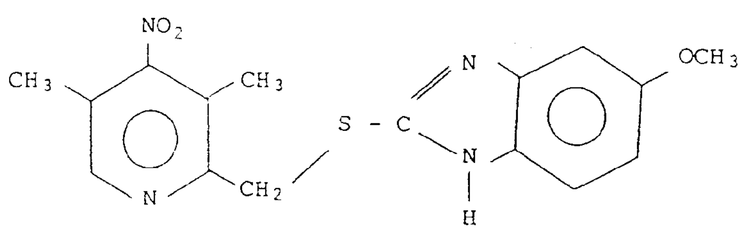

(VII)

and d) nucleophilic substitution of the $NO_2$ group by the-$OCH_3$ radical, which by treatment with a carboxylic acid, leads to the corresponding 5-methoxy-2-((3,5-dimethyl-4-methoxy-2-pyridinyl)methylthio)-1H-benzimidazoyl carboxylate of formula VIII

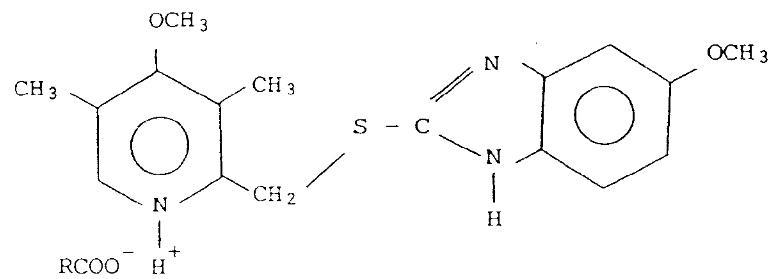

(VIII)

where RCOOH is an alkylcarboxylic acid, with the oxidation of the formula VIII compound leading to omeprazol of formula I.

B:

a) allyl chlorination of the 2-position methyl group, in the compound of formula III by reaction with a halogenating agent, to obtain a compound of formula IX

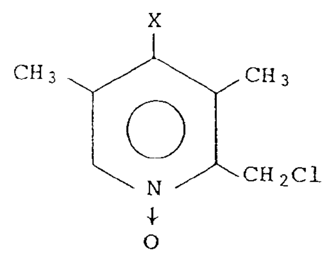

(IX)

where X is a halogen; b) nucleophilic substitution of the -Cl radical of the 2-position methyl group by the -SR radical, where R has the meaning given above, by reaction of the compound of formula IV with 5-methoxy-2-mercapto-benzimidazole of formula V to give 5-methoxy-2-((3,5-di-methyl-4-halo-2-pyridinyl-N-oxide)methylthio)-1H-benzimidazole of formula X

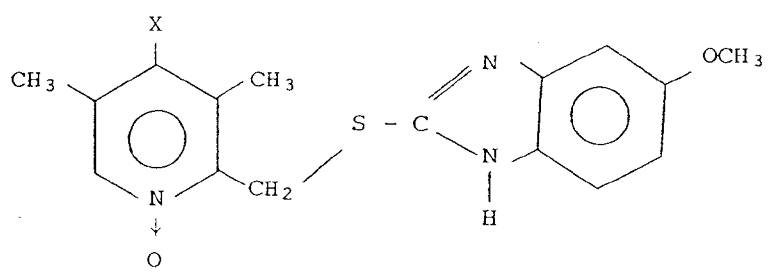

(X)

c) deoxygenation of the compound of formula X by reaction with a reducing compound to give 5-methoxy-2-((3,5-dimethyl- 4-halo-2-pyridinyl)methylthio)-1H-benzimidazole of formula XI

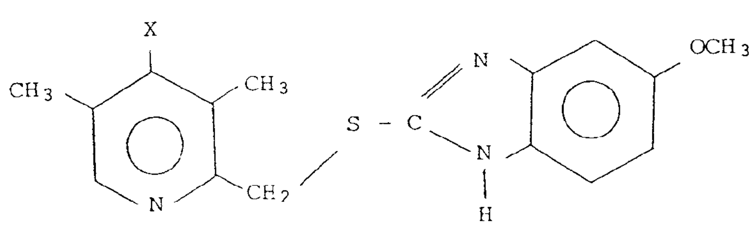

(XI)

and d) nucleophilic substitution of the halogen group by the radical $-OCH_3$, which when treated with a carboxylic acid gives the corresponding 5-methoxy-2-((3,5-dimethyl-4-methoxy-2-pyridinyl)methylthio)-1H-benzimidazoyl carboxylate of formula VIII, oxidation of which leads to omeprazol of formula I.

C:

a) allyl chlorination of the 2-position methyl group, after neutralization of said salt of a nitrated compound by treatment with a halogenating agent, to give a compound of formula IV; b) nucleophilic substitution of the -Cl radical of the 2-position methyl group by the -SR radical, where R has the meaning given above, by reaction of the compound of formula IV with 5-methoxy-2-mercapto-benzimidazole of formula V to give 5-methoxy-2-((3,5-dimethyl-4-nitro-2-pyridinyl-N-oxide)methylthio)-1H-benzimidazole of formula VI; c) nucleophilic substitution of the $NO_2$ group by the radical-$OCH_3$, which leads to 5-methoxy-2-((3,5-dimethyl-4-methoxy-2-pyridinyl-N-oxide)methylthio)-1H-benzimidazole of formula XII

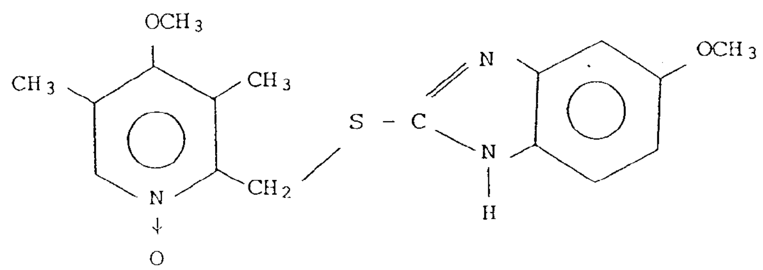

(XII)

and d) deoxygenation of the compound of formula XII, by reaction with a reducing compound, which when treated with a carboxylic acid gives the corresponding 5-methoxy-2-((3,5-dimethyl-4-nitro-2-pyridinyl)methylthio)-1H-benzimidazoyl carboxylate of formula VIII, oxidation of which leads to omeprazol of formula I.

D:

a) allyl chlorination of the 2-position methyl group, of a compound of formula III by reaction with a halogenating agent, to give a compound of formula IX; b) nucleophilic substitution of the -Cl radical of the 2-position methyl group, by the -SR radical, where R has the meaning given above, by reaction of the compound of formula IX with 5-methoxy-2-mercapto-benzimidazole of formula V to give 5-methoxy-2-((3,5-dimethyl-4-halo-2-pyridinyl-N-oxide)-methylthio)-1H-benzimidazole of formula X; c) nucleophilic substitution of the halogen by the radical -$OCH_3$ , which leads to 5-methoxy-2-((3,5-dimethyl-4-methoxy-2-pyridinyl-N-oxide)methylthio)-1H-benzimidazole of formula XII and d) deoxygenation of the compound of formula XII, by reaction with a reducing compound, which when treated with a carboxylic acid gives the corresponding 5-methoxy-2-((3,5-dimethyl-4-nitro-2-pyridinyl)methylthio)-1H-benzimidazoyl carboxylate of formula VIII, oxidation of which leads to omeprazol of formula I.

E :

a) allyl chlorination of the 2-position methyl group, after neutralization of said salt of a nitrated compound by reaction with a halogenating agent, to give a compound of formula IV; b) deoxygenation of the compound of formula IV, by reaction with a reducing compound to give 2-chloromethyl-3,5-dimethyl-4-nitro-pyridine hydrochloride of formula XIII

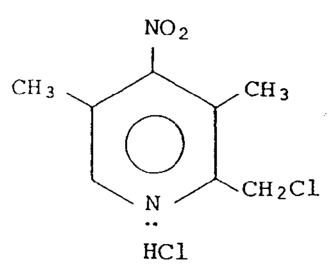

(XIII)

c) nucleophilic substitution of the -Cl radical of the 2-position methyl group, with the -SR radical, where R has the meaning given above, by reaction of the compound of formula XIII with 5-methoxy-2-mercapto-benzimidazole of formula V, to give the compound of formula VII; and d) nucleophilic substitution of the $NO_2$ group by the -$OCH_3$ radical which by treatment with a carboxylic acid gives the corresponding 5-methoxy-2-((3,5-dimethyl-4-methoxy-2-pyridinyl)methylthio)-1H-benzimidazoyl carboxylate of formula VIII, oxidation of which gives the omeprazol of formula 1.

F:

a) allyl chlorination of the 2-position methyl group, of a compound of formula III by reaction with a halogenating agent, to give the compound of formula IX; where X is a halogen; b) deoxygenation of the compound of formula IX by reaction with a reducing compound, to give the 2-chloromethyl-3,5-dimethyl-4-halo-pyridine hydrochloride of formula XV

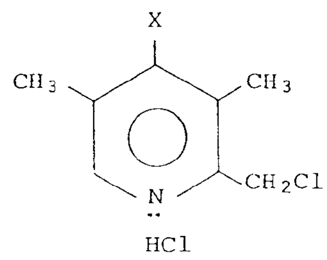

(XV)

c) nucleophilic substitution of the -Cl radical of the 2-position methyl group, by the -SR radical, where R has the meaning given above, by reaction of the compound of formula XV with 5-methoxy-2-mercapto-benzimidazole of formula V, to give the compound of formula XI; and d) nucleophilic substitution of the halogen group by the -$OCH_3$ radical which by treatment with the carboxylic acid gives the corresponding 5-methoxy-2-((3,5-dimethyl-4-methoxy-2-pyridinyl)methylthio)-1H-benzimidazoyl of formula VIII, oxidation of which gives the omeprazol of formula I.

Hereafter further alternatives of less interest than those considered above are give. These are:

G :

a) deoxygenation, after neutralization, of said salt of a nitrated compound by reaction with a reducing compound to give 2,3,5-trimethyl-4-nitro-pyridine of formula XVII

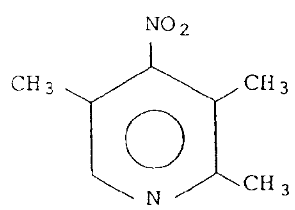

(XVII)

b) allyl chlorination of the 2-position methyl group, of the compound of formula XVII by reaction with a halogenating agent to give 2-chloromethyl-3,5-dimethyl-4-nitro-pyridine of formula XVIII

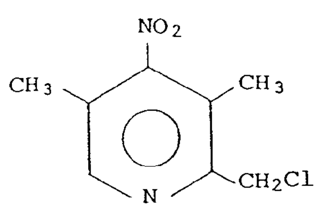

(XVIII)

c) nucleophilic substitution of the -Cl radical by the -SR radical, where R has the meaning given above, by reaction of the compound of formula XVIII with 5-methoxy-2-mercapto-benzimidazole of formula V, to give 5-methoxy-2-((3,5-dimethyl-4-nitro-2-pyridinyl)methylthio-1H-benzimidazole of formula VII; and d) nucleophilic substitution of the $NO_2$ group by the radical -$OCH_3$, which by treatment with a carboxylic acid gives the corresponding 5-methoxy-2-((3,5-dimethyl-4-methoxy-2-pyridinyl)methylthio)-1H-benzimidazoyl carboxylate of formula VIII, oxidation of which gives the omeprazol of formula I.

H :

a) deoxygenation of a compound of formula III by reaction with a reducing compound, to give 2,3,5-trimethyl-4-halo-pyridine of formula XIX

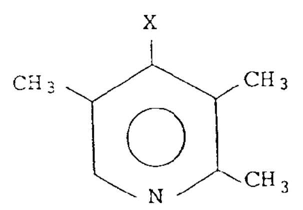

(XIX)

b) allyl chlorination of the 2-position methyl group, of the compound of formula XIX by reaction with a halogenating agent, to give 2-chloromethyl-3,5-dimethyl-4-nitro-pyridine of formula XX

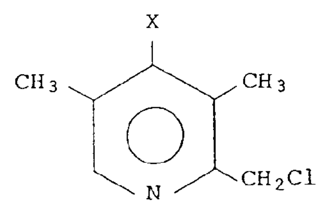

(XX)

c) nucleophilic substitution of the -Cl radical of the 2-position ethyl group, by the -SR radical, where R has the meaning given above, by reaction of the compound of formula XX with 5-methoxy-2-mercapto-benzimidazole of formula V, to give 5-methoxy-2-((3,5-dimethyl-4-halo-2-pyridinyl)methylthio-1H-benzimidazole of formula XI; and d) nucleophilic substitution of the halogen group by the $-OCH_3$ radical, which by treatment with a carboxylic acid gives the corresponding 5-methoxy-2-((3,5-dimethyl-4-methoxy-2-pyridinyl)methylthio)-1H-benzimidazoyl carboxylate of formula VIII, oxidation of which gives the omeprazol of formula I.

I:

a) nucleophilic substitution of the $NO_2$ group by the $-OCH_3$ radical in said salt of a nitrated compound by reaction with sodium methoxide, to give an intermediate which is not isolated; b) allyl chlorination of the 2-position methyl group, after neutralization of said intermediate, by an halogenating agent to give the 2-chloromethyl-3,5-dimethyl-4-nitro-pyridine N-oxide of formula XXII

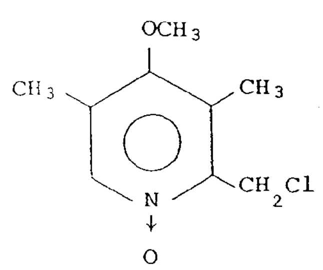

(XXII)

c) nucleophilic substitution of the -Cl radical by the -SR radical where R has the meaning given above by reaction of the compound of formula XXII with 5-methoxy-2-mercapto-benzimidazole of formula V to give the compound of formula XXIII

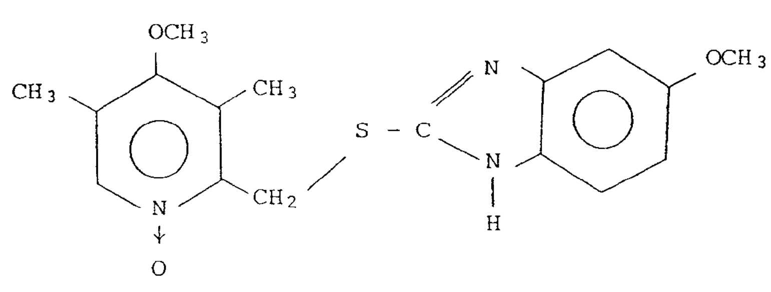

(XXIII)

d) deoxygenation of the compound of formula XXIII by reaction with a reducing compound which by treatment with a carboxylic acid gives the compound of formula VIII, oxidation of which leads to omeprazol of formula I.

J:

a) nucleophilic substitution of the halogen group by the $-OCH_3$ radical, in a compound of formula III by reaction with sodium methoxide, to give an intermediate which is not isolated; b) allyl chlorination of the 2-position methyl group, of said intermediate, by a halogenating agent to give 2-chloromethyl-3,5-dimethyl-4-methoxypyridine N-oxide of formula XXII

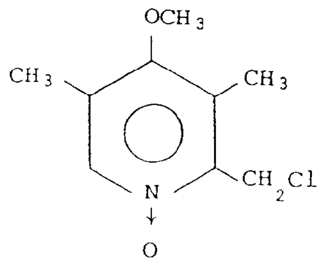

(XXII)

c) nucleophilic substitution of the -Cl radical by the -SR radical, where R has the meaning given above; by reaction of the compound of formula XXII with 5-methoxy-2-mercapto-benzimidazole of formula V to give the compound of formula XXIII

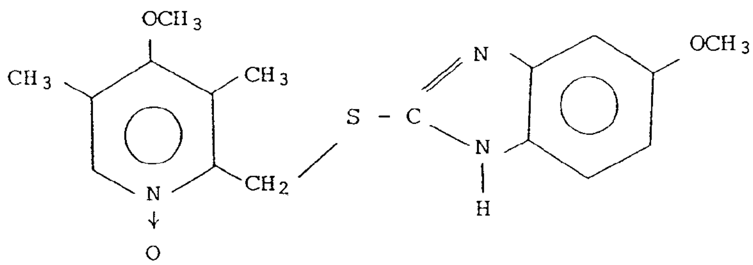

(XXIII)

d) deoxygenation of the compound of formula XXIII by reaction with a reducing compound, which after treatment with a carboxylic acid gives the compound of formula VIII, oxidation of which gives the omeprazol of formula I.

K:

a) nucleophilic substitution of the $NO_2$ group by the-$OCH_3$ radical, in said salt of a nitrated compound by reaction with sodium methoxide, to give an intermediate which is not isolated; b) allyl chlorination of the 2-position methyl group, of said intermediate, with an halogenating agent, to give 2-chloromethyl-3,5-dimethyl-4-methoxypyridine N-oxide of formula XXII

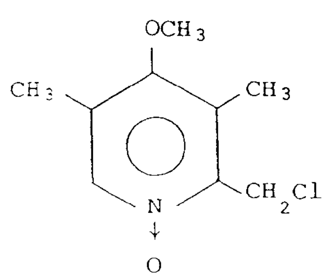

(XXII)

c) deoxygenation of the compound of formula XXII by reaction with a reducing compound to give 2-chloromethyl-3,5-dimethyl-4-methoxy-pyridine hydrochloride of formula XXIV

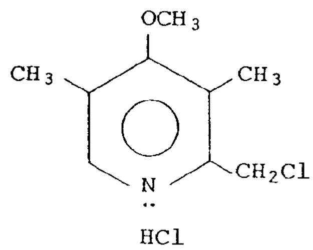

(XXIV)

d) nucleophilic substitution of the -Cl radical of the 2-position methyl group, by the -SR radical, where R has the meaning given above, by reaction of the compound of formula XXIV with 5-methoxy-2-mercapto-benzimidazole of formula V, which by treatment with a carboxylic acid gives the compound of formula VIII, oxidation of which gives the oeprazol of formula I.

L:

a) nucleophilic substitution of the halogen group by the $-OCH_3$ radical in a compound of formula III by reaction with sodium methoxide to give an intermediate which is not isolated; b) allyl chlorination of the 2-position methyl group, of said intermediate, with an halogenating agent, to give 2-chloromethyl-3,5-dimethyl-4-nitropyridine N-oxide of formula XXII; c) deoxygenation of the compound of formula XXII by reaction with a reducing compound to give 2-chloromethyl-3,5-dimethyl-4-methoxypyridine hydrochloride of formula XXIV; d) nucleophilic substitution of the -Cl radical of the 2-position methyl group, by the -SR radical, where R has the meaning given above, by reaction of the compound of formula XXIV with 5-methoxy-2-mercapto-benzimidazole of formula V, which by treatment with a carboxylic acid gives the compound of formula VIII, oxidation of which gives the omeprazol of formula I.

M:

a) O-acylation and subsequent acetoxylation of said salt of a nitrated compound, by means of acetic acid and acetic anhydride to give 2-acetoxymethyl-3,5-dimethyl-4-nitropyridine of formula XXV

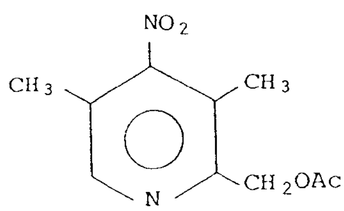

(XXV)

b) acid hydrolysis of the compound of formula XXV to give 2-hydroxymethyl-3,5-dimethyl-4-nitropyridine hydrochloride of formula XXVI

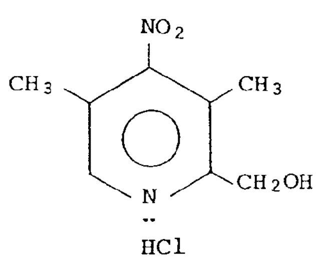

(XXVI)

c) nucleophilic substitution of the -OH radical by the -Cl radical by treatment with $SOCl_2$, to give 2-chloromethyl-3,5-dimethyl-4-chloropyridine hydrochloride of formula XXVII

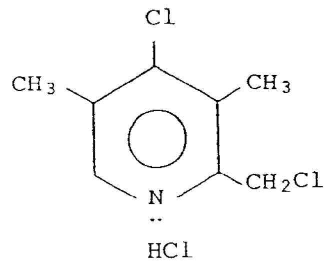

(XXVII)

d) nucleophilic substitution of the -Cl radical of the 2-position methyl group, by the -SR radical, where R has the meaning given above, by reaction of the compound of formula XXVII with 5-methoxy-2-mercapto-benzimidazole of formula V, to give the compound of formula XXVIII

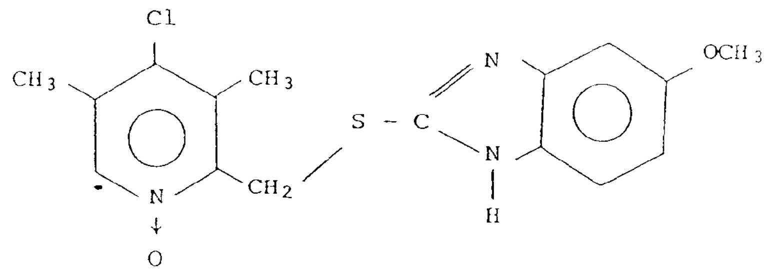

(XXVIII)

and e) nucleophilic substitution of the -Cl group by the $-OCH_3$ radical by reaction of the compound of formula XXVIII with sodium methoxide which, by treatment with a carboxylic acid, gives the compound of formula VIII, oxidation of which gives the omeprazol of formula I.

N :

a) O-acylation and subsequent acetoxylation of a compound of formula III with acetic acid and acetic anhydride to give 2-acetoxymethyl-3,5-dimethyl-4-halopyridine of formula XXIX

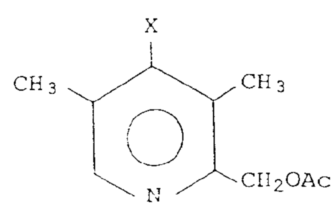

(XXIX)

b) acid hydrolysis of the compound of formula XXIX, giving 2-hydroxymethyl-3,5-dimethyl-4-halopyridine hydrochloride of formula XXX

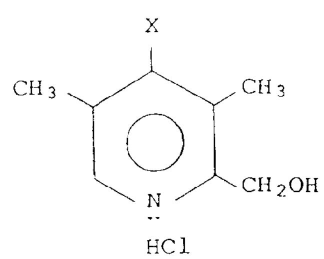

(XXX)

c) nucleophilic substitution of the -OH radical by the -Cl radical by treatment with a $SOCl_2$ to give 2-chloromethyl-3,5-dimethyl-4-halopyridine hydrochloride of formula XV; d) nucleophilic substitution of the -Cl radical of the 2-position methyl group, by the -SR radical, where R has the meaning given above, by reaction of the compound of formula XV with 5-methoxy-2-mercapto-benzimidazole of formula V, to give the compound of formula X; and e) nucleophilic subsitution of the halogen group by the - $OCH_3$ radical by the reaction of the compound of formula X with sodium methoxide which, by treatment with carboxylic acid, gives

the compound of formula VIII, oxidation of which gives the omeprazol of formula I.

The invention contemplates preferred features, with respect to the above alternatives, which are given below.

In alternatives A, C, E and G, the step a) neutralization is carried out by addition of an organic solvent over said salt of a nitrated compound and subsequent treatment with an aqueous alkali solution.

In alternatives A to F, the halogenating agent is sulfuryl chloride, in the presence of a catalyst (preferably Pd/tetraphenyl phosphine or benzoyl peroxide), or an N-chlorinated derivative of an amide or imide (preferably N-chlorosuccinimide, N-chlorobenzamide or trichloroisocyanuric acid).

In alternatives G and H, the halogenating agent is an N-chlorinated derivative of an amide or imide, the same as stated in the foregoing paragraph.

In the alternatives I to L, the halogenating agent is N-chlorosuccinimide.

In all the said alternatives, the reaction with 5-methoxy-2-mercapto-benzimidazole of formula V is carried out in an organic solvent, preferably ethanol, dimethyl sulfoxide, sulfolane, dimethyl acetamide or mixtures thereof, in the presence of sodium methoxide and optionally in presence of catalytic amounts of quaternary ammoniums, crown ethers or quaternary phosphoniums. The nucleophilic reactant of this reaction is the sodium or quaternary ammonium salt of the compound of formula V.

In alternatives A to L, the reducing agent is phosphorous trichloride, phosphorous tribromide, sulfur dichloride or sulfurous anhydride; the amounts of reducing agent used are stoichiometric or in a slight excess, and the reaction temperatures range from -40°C to room temperature.

In alternatives A to H, the nucleophilic substitution of the pyridine ring 4-position substituent is carried out by addition of sodium methoxide in the presence of a solvent (such as dimethylsulfoxide, sulfolane, N,N-dimethylacetamide or mixtures thereof or with ethanol), and of a catalyst (such as a quaternary ammonium salt, a quaternary phosphonium salt or a crown ether) such that the nucleophilic reactant of said substitution is the free anion $CH_3O^-$.

In alternatives A, C, E, G and I to M the nucleophilic substitution of the pyridine ring 4-position substituent is carried out by addition of sodium methoxide in the presence of methanol and a catalyst.

In alternatives M and N, the O-acylation and subsequent acetoxylation is carried out by addition of acetic acid and acetic anhydride in the presence of a catalyst (such as a 4-dialkylaminopyridine, e.g. 4-dimethylaminopyridine, 4-pyrrolidinepyridine or 4-guanidinepyridine) at a temperature ranging from 80 to 120 °C.

In alternatives M and N the acid hydrolysis is carried out with an aqueous inorganic acid, preferably hydrochloric acid and the nucleophilic substitution of the -OH radical by the-Cl radical is carried out by addition of thionyl chloride in the presence of methylene chloride as solvent and of a catalyst, such as an amide, preferably dimethylformamide. In all the alternatives A to N, the alkylcarboxylic acid of which the compound of formula VIII is a salt, is formic, acetic, monochloroacetic, dichloroacetic, pivalic, propanoic or 2-ethylhexanoic acid. The formula VIII salt is formed in situ in the substrate solution in a solvent, in which the salt is at least partly soluble. Preferably this solvent is ethyl, methyl or isopropyl acetate, methyl tert.butyl ether, 2,2-dimethoxypropane, acetone or methanol.

In all the alternatives A to N, the oxidation of the compound of formula VIII to omeprazol is carried out at temperatures ranging from -40 to 5°C with a peracid (preferably methachloroperbenzoic acid) except when the solvent is methanol. Once the oxidation is completed, the system is stabilized by addition of an organic base, preferably triethylamine.

When the solvent is methanol, the oxidation of compound VIII is carried out at a temperature ranging from -10 to 15°C with $H_2O_2$ in the presence of an inorganic alkaline base (preferably a carbonate) and of a catalyst (such as phosphotungstic acid, having formula $H_3$ $(P(W_3O_10)_4).xH_2O$, ammonium molybdate of formula $(NH_4)_2MoO_4$ , sodium tungstate of formula $Na_2WO_4$; phosphomolybdic acid of formula $H_3$ $(P\ (MO_3O_10)_4).xH_2O$; and silicotungstic acid, of formula $H_4$ $(Si(W_3O_10)_4).xH_2O$.

In all the alternatives A to N, the omeprazol of formula I obtained is recrystallized from an organic solvent (preferably ethyl acetate or acetone) or mixtures thereof, always in the presence of an organic base such as triethylamine.

To summarize, the new synthesis routes described in this patent, as well as the new reaction conditions used in several steps, allow a substantial reduction of a number of operative stages (where such stages have the meaning given before) involved in the conversion of 2,3,5-trimethylpyridine into omeprazol of formula I, also increasing the overall synthesis yield.

Thus the new reaction medium used for the N-oxidation of 2,3,5-trimethylpyridine (stage a′), and, to be precise, the substitution of the peracetic acid by one of the aforementioned percatalysts as oxidizing agent, has allowed the reaction time to be reduced from 30 hours to 70 minutes. The yield is practically quantitative.

According to one feature of the present invention, the N-oxide formed in stage a′) does not need to be isolated for nitration, stage f′), but this is carried out directly in situ after elimination of the excess oxidant and of

water, stages b') to e'). The said stage f') is carried out by new nitronium salts not used up today in nitration of pyridine systems, leading to almost quantitative yields.

According to a further feature of the present invention, the salt of the nitrated compound obtained in stage f') is converted into omeprazol in 5 to 7 chemical stages, the first of which is preferably a direct allylhalogenation of the methyl group adjacent the N-oxide group, preceded or otherwise by the substitution of the $-NO_2$ radical by a halogen, alternatives A to F. In all cases there is a great reduction of operative stages (where these have the meaning given before) relative to the synthesis method described in the literature. As an example, the alternative C is illustrated hereafter. For greater ease of reference with the scheme given on page 6, the initial compound of the present scheme is also 3 chemical steps from 2,3,5-trimethylpyridine.

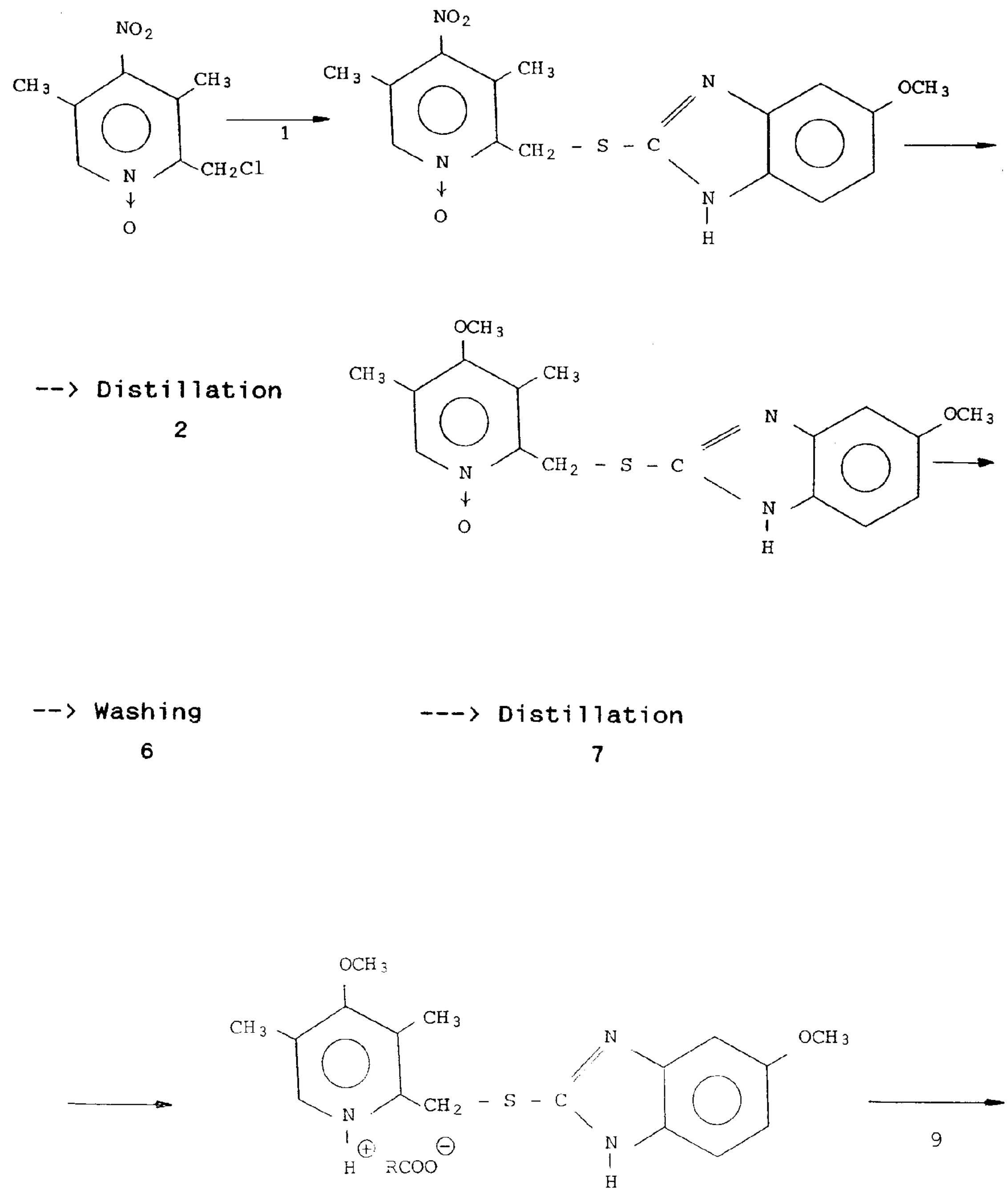


Omeprazol (white). Purity > 99%

In the preferred synthesis sequences, alternatives A to F, the allyl halogenation of the 2-methyl group, step 1, gives particularly satisfactory yields, in excess of 80%, when trichloroisocyanuric acid is used as the

halogenating agent. This reaction is based on the method described in "Chem. Ber.", 120, 649 (1987). That article describes that the treatment of various trimethylpyridines with trichloroisocyanuric acid allows the corresponding chlorinated derivatives in the 2-methyl group to be obtained, with yields of about 40%; in the case of 2-methylpyridine, the yield is substantially greater, 66%.

The use of trichloroisocyanuric acid for allyl chlorination of 2-methylpyridine N-oxides is not all obvious. Thus, for example, when 2,3,5-trimethyl-4-methoxypyridine N-oxide, formula CIII, is treated with said halogenating agent, the 6-chloro-2,3,5-trimethyl-4-methoxypyridine N-oxide of formula CIV is obtained with a good yield.

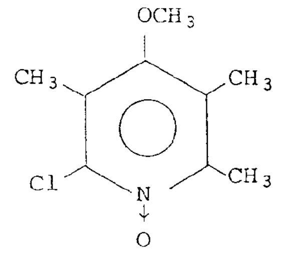

CIV

On the contrary, when the salt of the nitrated compound obtained in stage f') or the product of formula III is treated as above, the corresponding chlorinated derivatives in the 2-methyl group (compounds of formula IV and IX, respectively), are obtained with high yields.

The nucleophilic substitution of step 2) is carried out using the sodium or quaternary ammonium salt of 5-methoxy-2-mercapto-benzimidazole as nucleophile and evolves, under the conditions described (for the first time) herein, with practically quantitative yields in all cases.

The deoxygenation, step 3), is based on an application of the method described in "J. Chem. Soc., 1960, 4953. The maximum yields were obtained when reacting with $PCl_3$, using stochiometric amounts of the reactants and low temperatures (about -10 °C). Said yields vary between 50 and 90%, depending on the substrate. When testing this reaction with ammonium formate in MeOH in the presence of Pd/C as catalyst, following the method described in "Synthesis", no. 8, p. 645-646 (1989), negative results were obtained.

According to a feature of the present invention, the free anion $CH_3O^-$ is used as nucleophile in the nucleophilic substitution of step 4). The yields obtained in all cases are above 85%. It should be pointed out that when step 4) is carried out subsequently to step 2), said substitution is hindered, since the nucleophile must attack a negatively charged molecule. When the leaving group was nitrite, the free anion $CH_3O^-$ in MeOH allowed the nucleophilic substitution to be carried out in all cases. On the contrary, when the leaving group was a halogen, the reaction did not take place. This difficulty is solved by adding a cosolvent such as to allow an increase of the temperature.

The alternatives M and N represent a modification of the sequence of synthesis of omeprazol described in the literature. This modification is not, however, as obvious as it might seem since, apart from the difficulties mentioned in the foregoing paragraph, there should be added the fact that the initial O-acylation, step 1′) is carried out on substrates which are foreseeably much less activated for this reaction than the compound of formula CIII. Surprisingly the reaction proceeds with particularly high yields, in excess of 90%, when the acetic anhydride treatment is carried out in the presence of catalytic amounts of a 4-dialkylaminopyridine.

The acid hydrolysis of the acetoxy derivatives of formulas XXV and XXIX, step 2′), allows the hydrochlorides of formula XXVI and XXX to be prepared with yields of about 70% and high purity, which allows the notable loss of yield inherent in the reaction conditions reported in the literature and the inevitable subsequent distillation of the alcohol obtained, generating a large amount of resinous residue (see scheme on page 4), to be obviated.

Finally, step 3′) proceeds with high yields, in excess of 90%, when the thionyl chloride treatment is carried out in the presence of N,N-dimethylformamide.

According to a feature of the present invention, the final oxidation to omeprazol of formula I (alternatives A to N), is carried out with a peracid, preferably m-chloroperbenzoic acid, in an acid medium, except when the solvent is methanol. The acidity is known from the literature to hinder or prevent the N-oxidation reactions and the formation of sulfones, undesirable side effects in the preparation of omeprazol. Although these advantages are known, the use of acid conditions for the final S-oxidation to omeprazol was not foreseeable, since it is described in the literature that omeprazol is rapidly decomposed by acids; nevertheless, when operating with the salts of formula VIII, which are new in the literature, in appropriate solvents, said S-oxidation is not only possible but also leads to a white colour, good quality end product with good yields of around 95%. These salts also allow the starting thioether, of which compound VIII is a salt, to be stabilized against atmospheric oxygen in

storage.

A feature that such salts should have is that of being partly soluble in the solvent. Under these circumstances, the oxidation proceeds by way of a "salt solubilization - omeprazol precipitation" system, provided that the solvent is appropriate for such precipitation to take place. The solvents which have satisfied all these conditions (partial solubilization of the formula VIII salts and insolubilization of omeprazol) were various ethers and esters, preferably ethyl acetate. The reaction is very fast: the thin layer chromatographic analysis carried out 5 minutes after completing the addition of the oxidant shows a complete reaction.

When the reaction has terminated, the omeprazol formed is partially precipitated and partially dissolved in the form of the corresponding carboxylate (new salts in the literature). This salt is destroyed by the addition of an organic base, preferably triethylamine, which insolubilizes the omeprazol, allowing it to be isolated by simple filtration or centrifugation. This new method avoids, of course, all the problems raised by the isolation of omeprazol by distillation of the solvent described in the literature.

According to a further feature of the present invention, when the solvent is methanol, said final S-oxidation is carried out with hydrogen peroxide in the presence of a catalyst such as ammonium molybdate, having the formula $(NH_4)_2MoO_4$, and an inorganic base such as sodium carbonate. The omeprazol thus obtained may be isolated by extraction with $CH_2Cl_2$ , addition of an ester or a ketone, such as acetone, and of an organic base, such as triethylamine, and insolubilization of the omeprazol by fractional distillation of the $CH_2Cl_2$.

This method also allows omeprazol to be obtained with a good yield and high purity, with the advantage over the remaining methods which use peracids for this oxidation of allowing operation at temperature levels close to room temperature (energy saving) and, above all, of using a substantially cheaper compound, hydrogen peroxide, as oxidizing reactant.

Although the isolation of omeprazol requires a distillation operation to be carried out in this case, which is foreseeably long on an industrial scale, this should not raise any problem since it has been checked that a sample of pure omeprazol subjected to such treatent gives the starting product again with "yields" in excess of 90% and high purity, even after several hours at reflux.

According to a final feature of the present invention, the recrystallization of the omeprazol prepared according to any of the methods specified herein, using ethyl acetate or acetone in the presence of an organic base such as triethylamine, gives an omeprazol having a 99.7% (HPLC) -99.35% (perchloric acid analysis) purity and which is white and stable.

EXAMPLE 1

N-OXIDATION AND NITRATION

5 g (0.041 mole) of 2,3,5-trimethylpyridine, 0.087 g of $H_3 (P(W_3O_10)_4 ).xH_2O$, 0.1 ml of $CH_3SO_3H$ and 5 ml (0.0735 mole) of 50% $H_2O_2$ were mixed.

The system was heated to 90°C and held at 90-95°C for 70 minutes. At the end of this time the reaction was terminated (which was checked by thin layer chromatography). The system was cooled to room temperature.

The amount of 100% hydrazine hydrate required to reduce the excess $H_2O_2$ completely was added and thereafter the stoichiometric amount of fuming $HNO_3$ was added. The mixture was distilled at ordinary pressure, terminating under vacuum.

The moisture remains were destroyed (titrated according to Karl Fischer) by adding the necesarry amount of acetic anhydride and thereafter 15 ml of fuming $HNO_3$ and a further 5 ml of acetic anhydride were added.

The mixture was heated to reflux, was held for 30 minutes and then distilled at atmospheric pressure, terminating under vacuum.

A mixture of salts of the nitrated compound was obtained and was used as a starting product for the nucleophilic substitution reactions by methoxy (Example 8) or chlorine (Example 20) or for the allyl chlorination (Example 4).

EXAMPLE 2

N-OXIDATION AND NITRATION

25 g (0.207 mole) of 2,3,5-triethylpyridine, 0.435 g of $H_3 (P(W_3O_10)_4).xH_2O$, 0.5 ml of $CH_3 SO_3H$ and 10 ml (0.147 mole) of 50% $H_2O_2$ were mixed.

The system was heated to 90°C and held at 90-95°C for 2 hours 30 minutes, adding during this time a further three 5 ml portions of 50% $H_2O_2$ each (0.221 mole) after 5, 40 and 90 minutes reaction time.

When the reaction was terminated, the amount of hydrazine hydrate (51% anhydrous hydrazine) required for reduction of excess $H_2O_2$ was added at 90°C and thereafter the mixture was cooled to room temperature.

15 ml (0.357 mole) of fuming $HNO_3$ was added to the above mixture and thereafter the water was distilled at reduced pressure.

Thereafter 85 ml (2.023 mole) of fuming $HNO_3$ and 12 ml (0.1315 mole) of $O=PCl_3$ was added, the mixture was heated to 90°C and held at that temperature for 1 hour.

The operation continued as in Example 1.

EXAMPLE 3

4-CHLORO-2,3,5-TRIMETHYLPYRIDINE N-OXIDE

1 ml ($7.95.10^{-3}$ mole) of 2,3,5-trimethylpyridine, 0.5 g of $H_3$ $(P(W_3O_10)_4)$.$xH_2O$, and 1 ml (0.0147 mole) of 50% $H_2O_2$ were mixed.

The system was held at 90-95°C for 30 minutes, after which the end of the reaction was detected by chromatography.

The operation continued as in Examples 1 and 2.

EXAMPLE 4

2-CHLOROMETHYL-3,5-DIMETHYL-4-NITROPYRIDINE N-OXIDE

30% NaOH was added to the mixture of salts of nitrated compound produced by 6.70 g (0.055 mole) of 2,3,5-tri-methylpyridine to basic pH, followed by extraction with $CHCl_3$. The organic extracts were dried over anhydrous $Na_2SO_4$, were filtered and the solution was concentrated to about 65 ml.

0.2 g of benzamide were added followed by 14.0 g (0.060 mole) of trichloroisocyanuric acid. The mixture was heated to reflux and held at this temperature until complete disappearence in TLC of the spot attributable to the starting product.

It was filtered over celite and washed with methylene chloride. 50 ml of water were added and it was adjusted to basic pH with 25% NaOH.

The mixture was decanted, the aqueous phase was extracted with methylene chloride and the organic phase was washed with water. It was dried over anhydrous sodium sulfate, filtered and evaporated.

11.07 g of an oil crystallizing with difficulty were obtained, giving an orange colour pasty solid which turned pink on extended exposure to the light. Yield: 93%.

EXAMPLE 5

5-METHOXY-2-((3,5-DIMETHYL-4-NITRO-2-PYRIDINYL-N-OXIDE)-METHYLTHIO)-1H-BENZIMIDAZOLE

81 ml of methanol were added to 7 g (0.0323 mole) of 2-chloromethyl-3,5-dimethyl-4-nitropyridine N-oxide. 5.81 g (0.0323 mole) of 2-methoxymercaptobenzimidazole were added to this transparent yellow solution, giving an intense yellow suspension which was heated to reflux.

When a temperature of 35°C was reached, a solution of 1.33 g (0.0323 mole) of 98% NaOH and 8.5 ml of $H_2O$ was added over 19 minutes, while heating was continued up to reflux. At the end of the addition the pH was 8.

The system was held for 1 hour 30 minutes under reflux, the final pH being 7.

It was cooled to 35°C and 161 ml of $H_2O$ were added, with separation of a brown oil. Ethyl acetate was added followed by extraction and decantation. Thereafter the aqueous phases were extracted with ethyl acetate. Finally the pooled organic phases were washed with NaCl saturated water.

The organic phases were anhydrified with anhydrous $Na_2SO_4$, filtered and evaporated under vacuum, giving 11.6 g of a yellow solid product which means a yield of 100%.

Melting point 65-70 °C.

EXAMPLE 6

5-METHOXY-2((3,5-DIMETHYL-4-NITRO-2-PYRIDINYL)-METHYLTHIO)-1H-BENZIMIDAZOLE

2.0 g (0.0146 mole) of phosphorus trichloride were added over 5 minutes over a solution of 3.3 g (9.2

mmoles) of 5-methoxy-((3,5-dimethyl-4-nitro-2-pyridinyl-N-oxide)methylthio)-1H-benzimidazole in 4 ml of chloroform cooled to -15°C, the temperature being held below -5°C.

The mixture was stirred for 1 hour, holding the temperature to about -10°C.

30 ml of water were added to the reaction mixture and the pH was adjusted to 8 with sodium carbonate. The mixture was extracted with $CHCl_3$, dried, filtered and evaporated. 2.2 g of a brown solid were obtained. Yield: 70%.

EXAMPLE 7

5-METHOXY-2((3,5-DIMETHYL-4-METHOXY-2-PYRIDINYL)-METHYLTHIO-1H-BENZIMIDAZOLE

4 ml (22 mmoles) of 30% sodium methoxide in methanol were added to a mixture of 1.2 g (3.49 mmoles) of 5-methoxy-2-((3,5-dimethyl-4-nitro-2-pyridinyl)methylthio)-1H-benzimidazole and 0.1 g of benzyltriethylammonium chloride. The temperature was raised to 60°C and held at this level for 1 hour.

The mixture was neutralized with glacial acetic acid, the solvent was evaporated off, water was added followed by extraction with methylene chloride. The mixture was dried, filtered and evaporated. 1.1 g of a brown solid were obtained.

Yield: 96%.

EXAMPLE 8

5-METHOXY-2((3,5-DIMETHYL-4-METHOXY-2-PYRIDINYL-N-OXIDE)-METHYLTHIO)-1H-BENZIMIDAZOLE

100 ml of methanol and 0.5 g ($2.19.10^{-3}$ mole) of benzyltriethylammonium chloride were added to 10 g (0.0277 mole) of 5-methoxy-2-((3,5-dimethyl-4-nitro-2-pyridinyl-N-oxide)methylthio)-1H-benzimidazole. An ochre colour suspension was obtained.

The mixture was heated to reflux and once the system was boiling, 14.8 ml (0.0833 mole $CH_3ONa$) of a 30% MeONa solution in methanol was added over 33 minutes.

The system was then held at reflux (deep orange solution) for 1 hour 35 minutes.

4.5 g of benzyltriethylammonium chloride (0.0197 mole) plus 5 ml of methanol were added followed by 14.8 ml (0.0833 mole $CH_3ONa$) of 30% MeONa solution in methanol over 7 minutes.

The opalescent orange solution was held under reflux for a further 2 hours 35 minutes.

The system was cooled to room temperature and the pH was adjusted to 8 with glacial acetic acid.

The system was evaporated until an orange solid was obtained, to which $H_2O$ and $CH_2$ $Cl_2$ were added, followed by extraction and decantation. The aqueous phases were extracted with $CH_2Cl_2$.

The organic phases were anhydrified with anhydrous $Na_2SO_4$, filtered and evaporated under vacuum.

30 ml of ether ethyl were added to the amber oil obtained followed by further vacuum evaporation.

9.59 g of an ochre colour solid were obtained meaning a 100% yield. Pasty appearance.

EXAMPLE 9

5-METHOXY-2-(((3,5-DIMETHYL-4-METHOXY-2-PYRIDINYL)METHYL)-SULFINYL)-1H-BENZIMIDAZOLE

A solution of 1 g ($2.9.10^{-3}$ mole) of 5-methoxy-2-(((3,5-dimethyl-4-methoxy-2-pyridinyl)methyl)sulfinyl-1H-benzimidazole N-oxide in 2 ml of $CHCl_2$ was cooled to -20°C and 0.4 ml ($4.6.10^{-3}$ mole) of $PCl_3$ was added in a few seconds at a temperature ranging from -20 to -8°C.

The system was held for 90 minutes between -5 and 0°C.

10 ml of $H_2O$ were added to the reaction mixture and the pH was adjusted to 8 with $Na_2CO_3$ followed by extraction with $CHCl_3$.

The organic phase was dried with anhydrous $Na_2SO_4$, was filtered and evaporated, to give 0.74 g of an ochre colour solid. Yield: 78%. Melting point: 55-60°C.

EXAMPLE 10

5-METHOXY-2-(((3,5-DIMETHYL-4-METHOXY-2-PYRIDINYL)METHYL)-SULFINYL)-1H-BENZIMIDAZOLE

A solution of 2 g ($5.79.10^{-3}$ mole) of 5-methoxy-2-(((3,5-dimethyl-4-methoxy-2-pyridinyl)methyl)sulfinyl-1H-benzimidazole N-oxide in 4 ml of $CHCl_3$ was cooled to -15°C and 0.58 ml (6.33.10⁻3 mole) of $PCl_3$ was added in a few seconds at a temperature ranging from -15 to -6°C.

The system was held for 80 minutes between -5 and 0°C.

10 ml of $H_2O$ and 4 ml of $CHCl_3$ were added to the reaction mixture. The emulsion was adjusted to pH = 6 with $NaHCO_3$. It was extracted with $CH_2Cl_2$ and the organic phase was washed with $H_2O$.

The organic phase was dried with anhydrous $Na_2SO_4$, was filtered and evaporated, to give 1.67 g of an ochre colour solid. Yield: 88%. Melting point: 45-50°C.

EXAMPLE 11

5-METHOXY-2((3,5-DIMETHYL-4-METHOXY-2-PYRIDINYL)-METHYLTHIO)-1H-BENZIMIDAZOLE

2 g (5.80.10⁻ 3 mole) of 5-methoxy-2((3,5-dimethyl-4-methoxy-2-pyridinyl-N-oxide)-methylthio)-1H-benzimidazole were dissolved in 4 ml of chloroform. 0.58 ml (6.27.10⁻3 mole) of $PBr_3$ was added over 2 minutes to the opalescent deep orange solution cooled to 0°C. The reaction was very exothermic and the temperature was held between 0 and 8°C during the addition.

The system was held between 0 and 10°C for 1 hour. At the end of this time, 4 ml of chloroform and 10 ml of $H_2O$ were added at 0°C; the latter was added very slowly since the addition is very exothermic and the temperature is held between 0-10°C, acid pH.

$NaHCO_3$ was added to pH = 5. During the neutralization the temperature was held at 0°C.

$H_2O$ and chloroform were added, followed by extraction and decantation. The aqueous phase was extracted with chloroform and the organic phase was washed several times with $H_2O$.

The organic phases were anhydrified with anhydrous $Na_2SO_4$. The system was filtered and evaporated under vacuum.

1.17 g of a browny orange solid were obtained, representing a 62% yield.

EXAMPLE 12

2-CHLOROMETHYL-3,5-DIMETHYL-4-NITROPYRIDINE HYDROCHLORIDE

2.0 g (0.0146 mole) of phosphorus trichloride were added dropwise over 4 minutes to a solution of 2.0 g (0.0092 mole) of 2-chloromethyl-3,5-dimethyl-4-nitropyridine N-oxide in 4 ml of chloroform cooled to -10°C, holding the temperature below -5°C.

The mixture was stirred for 1 hour, while holding the temperature between -5 and +5°C, it was evaporated at room temperature, ethyl acetate was added and hydrogen chloride was blown through for about 3 minutes.

The mixture was filtered to give 1.46 g of a powdery white solid. Yield: 67%. Melting point: 124-127 °C.

EXAMPLE 13

5-METHOXY-2((3,5-DIMETHYL-4-NITRO-2-PYRIDINYL)-METHYLTHIO)-1H-BENZIMIDAZOLE

1.20 g (0,0051 mole) of 2-chloromethyl-3,5-dimethyl-4-nitropyridine hydrochloride and 0.91 g (0.0051 mole) of 2-mercapto-5-methoxybenzimidazole were suspended in 13 ml of methanol and 0.21 g (0.0051 mole) of NaOH dissolved in 1.5 ml of water were added.

The mixture was heated to reflux and in the meantime a further 0.21 g (0.0051 mole) of NaOH in 1.5 ml of water was added dropwise over about 5 minutes. Reflux was held for 100 minutes, the pH was adjusted to 8-9 with 1N NaOH and 25 ml of water were added, with the appearance of a yellow precipitate being observed.

The mixture was filtered, dissolved in methylene chloride and dried over anhydrous $Na_2SO_4$. The mixture was filtered and evaporated.

1.43 g of a pale yellow coloured spongy solid were obtained. Yield: 82%. Melting point: 80-90°C.

EXAMPLE 14

4-CHLORO-2-CHLOROMETHYL-3,5-DIMETHYLPYRIDINE N-OXIDE

0.1 g of benzamide and 4.16 g (0.0243 mole) of 4-chloro-2,3,5-trimethylpyridine N-oxide were dissolved in 12.5 ml of chloroform and thereafter 3.1 g (0.0133 mole) of trichloroisocyanuric acid were added.

The mixture was heated to reflux and held at that temperature for 5 hours, was filtered over celite and

washed with 2 x 25 ml of methylene chloride. 50 ml of water were added to the filtrate and the pH was basified with 25% aqueous NaOH.

The mixture was decanted, the aqueous phase was extracted with methylene chloride and the organic phase was dried over anhydrous sodium sulfate.

The mixture was filtered and evaporated, to give 4.13 g of a whitish solid. Yield: 83%. Melting point: 134-141°C.

EXAMPLE 15

4-CHLORO-2-CHLOROMETHYL-3,5-DIMETHYLPYRIDINE HYDROHLORIDE

10.0 g (0.0485 mole) of 4-chloro-2-chloromethyl-3,5-dimethylpyridine N-oxide were dissolved in 20 ml of chloroform and 10 ml of phosphorous trichloride were added over 4 minutes while holding the temperature below 25°C.

The mixture was stirred for 30 minutes at room temperature, was evaporated, ethyl acetate was added and hydrogen chloride was blown through for about 3 minutes.

The mixture was filtered to give 6.02 g of a powdery white solid. Yield: 55%.

EXAMPLE 16

5-METHOXY-2-((3,5-DIMETHYL-4-CHLORO-2-PYRIDINYL)-METHYLTHIO)-1H-BENZIMIDAZOLE

176.5 ml of methanol were added over 16 g (0.07064 mole) of 4-chloro-2-chloromethyl-3,5-dimethylpyridine hydrochloride to give a pale yellow solution. Thereafter 12.7 g (0.07064 mole) of 2-methoxymercaptobenzimidazole were added. A solution of 2.88 g of 98% NaOH (0.0706 mole) and 18.5 ml of $H_2O$ were added at one time to the suspension obtained at room temperature. The addition was exothermic and the temperature rose to 32°C.

The system was heated to reflux and when the temperature reached 35°C, a further solution of 2.88 g of 98% NaOH (0.0706 mole) and 18.5 ml of $H_2O$ were added over 20 minutes while the temperature reached reflux. When the addition was completed, the reflux was held for 55 minutes. (The pH one minute after completing the addition was 7 and at the end of the reflux time was 6).

The mixture was cooled to 35°C and 353 ml of $H_2O$ were added. A very pale pink solid precipitated out. It was filtered, washed with $H_2O$, heptane and hexane. A violet solid which was dried at room temperature under vacuum and afterwards under a hot air current was obtained.

23.37 g of dry product were obtained, representing a 99.2% yield. Melting point 78-85°C.

EXAMPLE 17

5-METHOXY-2-((3,5-DIMETHYL-4-METHOXY-2-PYRIDINYL)-METHYLTHIO)-1H-BENZIMIDAZOLE

10 ml of dimethylsulfoxide were added over 2 g ($6x10^{-}3$ mole) of 5-methoxy-2-((4-chloro-3,5-dimethyl-2-pyridinyl)methylthio)-1H-benzimidazole to give a violet solution which turned to yellow with a subsequent addition of 4 ml ($2410^{-}3$ moles) of 30% sodium methoxide solution in methanol.

The system was heated to 95-100°C over 10 minutes and held at this temperature for 1 hour. At the end of this time, it was cooled to room temperature and the excess sodium methoxide was neutralized with acetic acid, after the prior addition of 50 ml of $H_2O$.

The mixture was extracted with $CH_2Cl_2$ over this suspension and decanted.

Finally, the $CH_2Cl_2$ phases were washed with water.

The organic phases were anhydrified with $Na_2SO_4$, filtered and evaporated.

1.77 g of product, a beige colour solid, representing 90% yield, were obtained.

Melting point: on making the preparation, the product was placed on the slide and when the cover was placed and observed through the microscope, an odd drop was observed. On heating to 50°C further drops appeared in the range of 40-50°C, but at no time was the sharp appearance of drops indicating a melting point observed.

Following the same method described in this example, but holding the system for 2 hours at 95-100°C, the yield drops to 84%.

Following the same method but holding the system for 3 hours at 75-70°C, a mixture of the starting and end products is obtained.

EXAMPLE 18

NUCLEOPHILIC SUBSTITUTION WITH METHOXIDE

A 30% sodium methoxide solution in methanol was added over the mixture of salts of nitrated compound from 3.66 g (0.030 mole) of 2,3,5-trimethylpyridine until pH = 5.

0.5 g of benzyltriethylammonium chloride was added to the resulting solution and thereafter 15 ml of 30% sodium methoxide in methanol was added over 35 minutes, holding the temperature to about 30°C.

The system was held between 30 and 50°C for 8 hours, the pH was adjusted to 11 with glacial acetic acid, the methanol was evaporated off and water was added to the solid obtained, followed by extraction.

It was dried over anhydrous $Na_2SO_4$ and filtered. The solution obtained was used directly for the allyl halogenation stage (example 19).

EXAMPLE 19

2-CHLOROMETHYL-3,5-DIMETHYL-4-METHOXIPYRIDINE N-OXIDE

The solution obtained in example 18 was concentrated to about 25 ml and 8.0 g (0.060 mole) of N-chlorosuccinimide were added and the temperature was held at reflux until the spot attributable to the starting N-oxide had disappeared in TLC, with the observation of the presence of a single spot elutable principally to a higher $R_f$ (eluant: $CHCl_3$/MeOH 15:1).

EXAMPLE 20

4-CHLORO-2,3,5-TRIMETHYLPYRIDINE N-OXIDE

3.2 g (0.039 mole) of sodium acetate and 5 ml of acetic acid were added to the mixture of salts of the nitrated compound obtained from 5 g (0.041 mole) of 2,3,5-trimethylpyridine. The system was stirred for 30 minutes at about 40°C.

21 ml (0.2943 mole) of acethyl chloride were added to the above suspension over about 15 minutes at a temperature ranging from 25 to 30°C.

The system was held for 2 hours 15 minutes at 30-35°C and then evaporated.

$H_2O$ was added to the crude product obtained and the mixture was adjusted to pH = 7 with a $Na_2CO_3$ It was extracted with $CH_2Cl_2$. The organic phase was dried with anhydrous $Na_2SO_4$, was filtered and evaporated, giving 6.9 g of a cream coloured solid, representing a practically quantitative yield. Melting point: 130-140°C.

EXAMPLE 21

2-ACETOXYMETHYL-3,5-DIMETHYL-4-NITROPYRIDINE

A solution of 24.3 ml (0.2570 mole) of acetic anhydride and 0.09 g ($7.10^{-4}$ mole) of 4-dimethyl-aminopyridine was prepared and heated to 90°C.

A solution of 15 g (0.0824 mole) of 2,3,5-trimethyl-4-nitropyridine and 8.3 ml (0.1451 mole) of acetic acid were added over about 25 minutes to the previous solution at a temperature of 90-100°C.

The system was held at 90-95°C for 1 hour, was then allowed to cool to 75°C and 35 ml of MeOH were added over a period of about 5 minutes.

Finally the system was evaporated to obtain a dense brown oil (18.28 g) with a 99% yield.

EXAMPLE 22

2-HYDROXIMETHYL-3,5-DIMETHYL-4-NITROPYRIDINE HYDROCHLORIDE

A mixture of 18 g (0.08 mole) of 2-acetoxymethyl-3,5-dimethyl-4-nitropyridine, 16 ml of $H_2$ and 42 ml (0.475 mole) of 35% HCl was prepared.

The system was held for 19 hours at room temperature under stirring, was then evaporated to dryness and 100 ml of isopropanol were added to the solid obtained.

After holding the system under stirring for about 15 minutes at room temperature, the suspended solid was filtered to give 12.09 g of the title compound (cream colour). Yield: 70%.

Melting point: 132-135°C.

EXAMPLE 23

4-CHLORO-2-CHLOROMETHYL-3,5-DIMETHYLPYRIDINE

A solution of 9.32 g (0.051 mole) of 2-hydroxymethyl-3,5-dimethyl-4-nitropyridine, 15 ml of dichloromethane and 3.5 ml of N,N-dimethylformamide was prepared.

5.5 ml (0.075 mole) of $SOCl_2$ was added to the above solution over 10 minutes, allowing the temperature to raise to 65 °C.

The system was held at 55-60°C for 90 minutes and then cooled to room temperature. 50 ml of $CH_2Cl_2$ were added to the reaction mixture and all of this was poured over a solution of 16 g (0.15 mole) of $Na_2CO_3$ and 200 ml of $H_2O$.

The mixture was decanted and the aqueous phase was extracted with $CH_2Cl_2$. The organic phase was them washed several times with $H_2O$ and finally dried with anhydrous $Na_2SO_4$.

After removing the $CH_2Cl_2$ phase by evaporation, 9.85 g of an orange coloured oil were obtained. Practically quantitative yield.

EXAMPLE 24

2-ACETOXYMETHYL-4-CHLORO-3,5-DIMETHYLPYRIDINE

A solution of 20.6 g of 4-chloro-2,3,5-trimethylpyridine N-oxide in 17 ml of acetic acid heated to 60°C was added over a period of about 15 minutes to a solution of 0.12 g of 4-dimethylaminopyridine in 35.4 ml of acetic anhydride held at 90°C.

When the addition was completed, the mixture was held at 95-105°C for 1 hour, was cooled to about 50°C and 56 ml of methanol were added over about 15 minutes, holding the temperature to below about 65 °C. The mixture was evaporated under vacuum to give 23.74 g of a brown oil which partially crystallized. Yield: 93%.

EXAMPLE 25

4-CHLORO-2-HYDROXYMETHYL-3,5-DIMETHYLPYRIDINE HYDROHLORIDE

A solution of 56 ml of 35% hydrochloric acid (0.633 mole) and 44 ml of $H_2O$ was added over 23 g (0.107 mole) of 2-acetoxymethyl-4-chloro-3,5-dimethylpyridine. The system was heated and held at reflux for 2 hours.

0.5 g of activated carbon was added and was allowed to rest for some time at room temperature, followed by filtering the carbon over Celite.

The orange coloured transparent solution was evaporated under vacuum.

100 ml of isopropyl alcohol were added to the product obtained to destroy moisture. A beige coloured suspension was obtained, was filtered and was washed with ethyl acetate.

A white solid which was dried by a hot air flow was obtained.

Yield about 74% on obtaining 16.64 g of end product. Melting point: 192-200°C.

EXAMPLE 26

4-CHLORO-2-HYDROXYMETHYL-3,5-DIMETHYLPYRIDINE HYDROCHLORIDE

A solution of 52 ml of 35% hydrochloric acid (0.588 mole) and 41 ml of $H_2O$ was added over 21.6 g (0.1011 mole) of 2-acetoxymethyl-4-chloro-3,5-dimethylpyridine. The system was heated and held at boiling for 3 hours 15 minutes.

It was allowed to cool to room temperature and evaporated. 150 ml of ethyl acetate were added over the yellow product obtained. It was stirred for a time at room temperature. The suspension obtained was filtered and washed with ethyl acetate, and was allowed to dry under vacuum at room temperature.

14.87 g of a yellow solid were obtained with a yield of 70.6%. Melting point: 186-190°C.

EXAMPLE 27

4-CHLORO-2-CHLOROMETHYL-3,5-DIMETHYLPYRIDINE HYDROCHLORIDE

23 ml of $CH_2Cl_2$ and 0.8 ml (0.0103 mole) of dimethylformamide were added over 16 g (0.0769 mole) of 4-chloro-2-hydroxymethyl-3,5-dimethylpyridine. 8.2 ml of thionyl chloride (0.1135 mole) were added over the suspension in 9 minutes at room temperature with stirring. The reaction was exothermic and temperature was held between 25-30°C during the addition.

The system was held for 2 hours 30 minutes at room temperature and thereafter held at reflux for 20 minutes.

4.5 ml of $CH_2Cl_2$ were distilled (internal temperature 45°C) and 45.5 ml of ethyl acetate were added to give a yellow coloured suspension. It was cooled to 20-25°C, was filtered and washed with ethyl acetate. It was allowed to dry under vacuum at room temperature.

16.61 g of white solid were obtained, representing a 95% yield. Melting point: 142-154°C.

EXAMPLE 28

OMEPRAZOL

A mixture of 4 g (0.0117 mole) of 5-methoxy-2-(((3.5-dimethyl-4-methoxy-2-pyridinyl)methyl)-sulfinyl)-1H-benzimidazole of 96% purity was prepared in 25 ml of ethyl acetate and 1.7 ml (0.0103 mole) of 2-ethylhexanoic acid.

The mixture was cooled to -10°C and 3.06 g (0.0121 mole) of 68% pure m-chloroperbenzoic acid were added over about 25 minutes at a temperature ranging from -10 to -5°C.

The system was held at -10°C for 15 minutes after which it was cooled to -40°C and 1.8 ml (0.013 mole) of triethylamide were added without allowing the temperature to rise above-30°C.

The system was held for 20 minutes at -40°C and the suspension was then filtered. The solid obtained was washed with 20 ml AcOEt at -20°C and dried under a current of air at room temperature, giving a weight of 3.5 g. Yield: 89% (snow white colour). Melting point: 147-150°C.

EXAMPLE 29

OMEPRAZOL

A solution of 25 ml ethyl acetate and 1.7 ml (0.0103 mole) of 2-ethylhexanoic acid was prepared, to which there were added 4 g (0.0117 mole) of 96% pure 5-methoxy-2-((3,5-dimethyl-4-methoxy-2-pyridinyl)methylthio)-1H-benzimidazole, 3.06 g (0.01214 mole) of metachloroperbenzoic acid were added to the resulting solution over 23 minutes at temperatures of -35 and -40°C.

When the addition was completed, the system was held -35°C for 1 hour.

Thereafter, 1.8 ml of triethylamine (0.013 mole) was added at that temperature after which the temperature was held at-35°C for 20 minutes.

The mixture was filtered at that temperature and washed with cold ethyl acetate at a temperature of -20°C.

It was allowed to dry under flowing air at room temperature.

A white solid (3.7 g) was obtained, representing a 94% yield.

The melting point of the product was 147-150°C.

EXAMPLE 30

OMEPRAZOL

A solution of 7 g (0.0212 mole) of 5-methoxy-2-((3,5-dimethyl-4-methoxy-2-pyridinyl)methylthio)-1H-benzimidazole and a mixture of tert.butyl methyl ether and 2-ethylhexanoic acid were prepared.

The system was cooled to -35°C and 5.36 g (0.0212 mole) of metachloroperbenzoic acid were added over a period of 30 minutes, holding the above temperature.

The reaction mixture was held for 1 hour at the same temperature of -35°C.

It was filtered at -5°C and washed with a few millilitres of tert.butyl methyl ether.

6.36 g of the title product were obtained, representing an 87% yield.

A practically white solid, having a melting point of 147-150°C.

EXAMPLE 31

OMEPRAZOL

A mixture of 4 g (0.0117 mole) of 96% pure 5-methoxy-2-(((3,5-dimethyl-4-methoxy-2-pyridinyl)methyl)-sulfinyl)-1H-benzimidazole in 25 ml of ethyl acetate and 0.6 ml (0.0105 mole) of acetic acid was prepared.

The mixture was cooled to -20°C and 3.06 g (0.0121 mole) of 68% pure m-chloroperbenzoic acid was added thereto over about 25 minutes at a temperature ranging from -20 to -25°C.

The system was held at -20°C for 5 minutes, after which it was cooled to -40°C and 3.4 ml (0.0246 mole) of triethylamine were added thereto, without the temperature rising above -30 °C.

The system was held for 20 minutes at -40 °C, and the suspension was then filtered. The solid obtained was washed with 20 ml of AcOEt at -20°C and was dried with flowing air at room temperature to give a weight of 3.5 go Yield: 89% (snow white colour). Melting point: 147-150 °C.

EXAMPLE 32

OMEPRAZOL

A mixture of 2 g (0.00607 mole) of 2-(((3,5-dimethyl-4-methoxy-2-pyridinyl)methyl)sulfinyl)-5-methoxy-1H-benzimidazole in 3 ml of methanol was prepared.

The mixture was cooled to about 10°C and a spatula tip (about 40 mg) of $Na_2CO_3$ was added.

The temperature was held and 0.45 ml of 50% $H_2O_2$ (0.00661 mole) was added dropwise followed by 20 mg of $(NH_4)_2MoO_4$.

The system was held for 2 hours and 15 minutes at a temperature ranging from 0 to 10°C.

At the end of this time, the reaction mixture was taken to room temperature and 5 ml of $H_2O$ were added. It was extracted with $CH_2Cl_2$.

The organic phase was dried with anhydrous $Na_2SO_4$ and evaporated at room temperature.

A whitish solid weighing 2.06 g was obtained, identified as omeprazol by thin layer chromatography and by IR.

Yield: 98%.

Melting point: 140-147°C.

EXAMPLE 33

OMEPRAZOL

A mixture of 2 g (0.00607 mole) of 2-(((3,5-dimethyl-4-methoxy-2-pyridinyl)methyl)sulfinyl)-5-methoxy-1H-benzimidazole in 3 ml of methanol was prepared. The mixture was cooled to 2°C and a spatula tip (about 40 mg) of $Na_2CH_3$ was added. Thereafter, while holding the temperature, 0.45 ml of 50% $H_2O_2$ (0.00661 mole) was added dropwise, followed by 20 mg of $(NH_4)_2MoO_4$.

The system was held for 2 hours between 0 and 5°C. 15 ml of ethyl acetate at 0°C were added over the suspension formed. The mixture was cooled to -15°C and filtered and washed with ethyl acetate at -15°C. 1.45 g of a white solid were obtained, identified as omeprazol by thin layer chromatography ($CHCl_3$/MeOH 15:1) and by IR.

Yield: 70%.

Melting point: 152-157°C.

## Claims

**1.-** A process for the preparation of omeprazol, the chemical name of which is 5-methoxy-2-(((3,5-dimethyl-4-methoxy-2-pyridinyl)methyl)sulfinyl)-1H-benzimidazole, of formula I

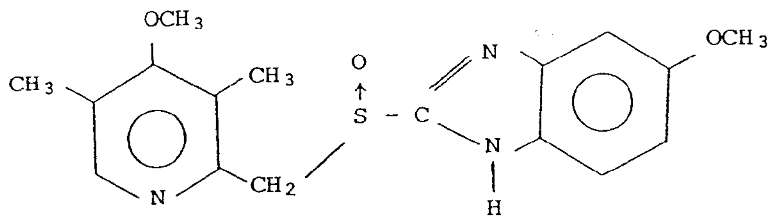

(I)

characterized by comprising the following stages: a) oxidation of 2,3,5-trimethylpyridine by an oxidizing agent formed by the reaction product of hydrogen peroxide and a catalyst, said oxidation taking place in the presence of catalytic traces of an acid selected from the group formed by the alkylsulfonic and arylsulfonic acids and sulfuric acid, to give N-oxide derivatives; b) reduction of the excess hydrogen peroxide to water by way of reducing agents; c) fixation of the N-oxide with an acid; d) distillation of the water until a low moisture content is obtained; e) addition of a dehydrating agent to destroy the said low moisture content; f) nitration by a nitronium salt of formula $A^{-}NO_2^{+}$, $A^{-}$ being acetate, a halogenide, a sulfonate or an oxyhalogenide of phosphorus oxidized in 3- or 5- position, to give a salt of a nitrated compound; and g) distillation of the excess nitric acid; and in that, optionally after a halogenation step giving a compound of formula III

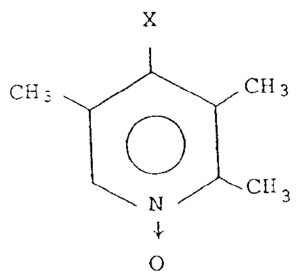

(III)

where X is a halogen; then the following takes place: either steps: 1) allyl chlorination of the 2-position methyl group; 2) nucleophilic substitution of the -Cl radical of the 2-position methyl group by the -SR radical, where R is

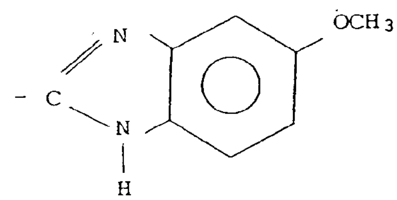

3) deoxygenation of the N-oxide; 4) nucleophilic substitution of the 4- position substituent by the $-OCH_3$ radical; and 5) oxidation providing omeprazol, such that steps 1) to 4) may take place in any order provided that step 2) is subsequent to step 1); and step 4) is either subsequent to step 2) or prior to step 1);

or, steps: 1′) O-acylation and subsequent acetoxylation of the 2-position methyl group; 2′) acid hydrolysis; 3′) nucleophilic substitution of the -OH radical by the -Cl radical; 4′) nucleophilic substitution of the -Cl radical of the 2-position methyl group by the -SR radical, where R has the meaning given above; 5′) nucleophilic substitution of the 4-position substituent by the $-OCH_3$ radical, after which an oxidation step providing the omeprazol is carried out.

**2.-** The process of claim 1, wherein the stage a) oxidation is carried out preferably at a temperature ranging from 30 to 100 °C.

**3.-** The process of claim 1, wherein said catalyst for stage a) is selected from the group formed by: phosphotungstic acid, having the formula $H_3$ $(P(W_3O_10)_4).xH_2O$; ammonium molybdate, having the formula $(NH_4)_2MoO_4$; sodium tungstate, having the formula $Na_2WO_4$; phosphomolybdic acid, having the formula $H_3$ $(P(Mo_3O_10)_4.xH_2O$; and silicotungstic acid, having the formula $H_4$ $(Si(W_3O_10)_4).xH_2O$.

**4.-** The process of claim 1, wherein said reducing agent of stage b) is preferably hydrazine hydrate, sulfurous anhydride, a sulfite, a bisulfite or a metabisulfite.

**5.-** The process of claim 1, wherein said acid of stage c) is a strong organic acid, nitric acid or sulfuric acid.

6.- The process of claim 1, wherein said stage e) dehydrating agent is preferably acetic anhydride, phosphorus oxychloride, phosphorus pentachloride or phosphorus trichloride.

7.- The process of claim 1, wherein said stage f) nitronium salt is formed in situ by addition over nitric acid of a compound selected from the group formed by a sulfonic acid, acetic anhydride and a phosphorus halogenide oxidized in 3- or 5- position.

8.- The process of claim 7, wherein said phosphorus halogenides are phosphorus oxychloride, phosphorus trichloride or phosphorus pentachloride, and said sulfonic acid 5-trifluoro-methanesulfonic acid or Nafion-H.

9.- The process of any one of claims 1 to 8, wherein the pyridine ring halogenation step is carried out by direct treatment of said salt of a nitrated compound, previously neutralized with sodium acetate and acetic acid, with X-CO-$CH_3$, $SOX_2$/ dimethylformamide, where X is halogen, at a temperature ranging from 0 to 55 °C, to give a compound of formula III.

10.- The process of any one of claims 1 to 9, comprising successively the following steps: a) allyl chlorination of the 2-position methyl group, after neutralization of said salt of a nitrated compound by the addition of a halogenating agent, to give a compound of formula IV

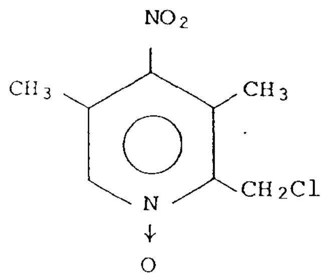

(IV)

b) nucleophilic substitution of the -Cl radical by the -SR radical, where R has the meaning given above, by reacting the compound of formula IV with 5-methoxy-2-mercapto-benzimidazole of formula V

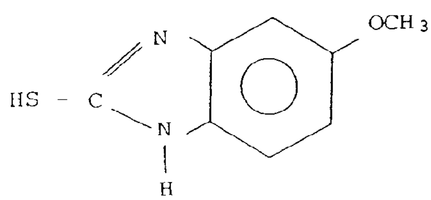

(V)

to give 5-methoxy-2-((3,5-dimethyl-4-nitro-2-pyridinyl-N-oxide)methylthio)-1H-benzimidazole of formula VI

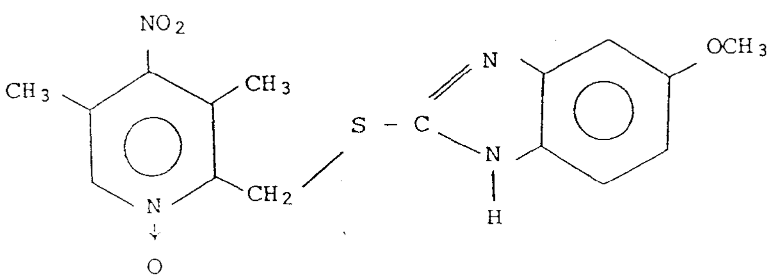

(VI)

c) deoxygenation of the formula VI compound by reaction with a reducing compound to give 5-methoxy-2-((3,5-dimethyl-4-methoxy-2-pyridinyl)methylthio)-1H-benzimidazole of formula VII

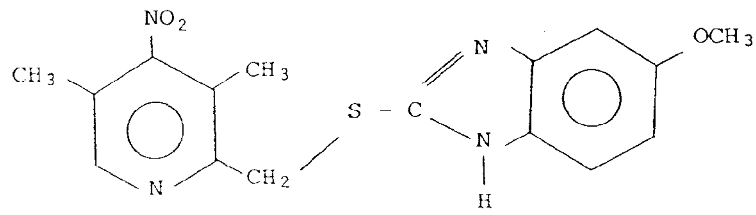

(VII)

and d) nucleophilic substitution of the $NO_2$ group by the-$OCH_3$ radical, which by treatment with a carboxylic acid, leads to the corresponding 5-methoxy-2-((3,5-dimethyl-4-methoxy-2-pyridinyl)methylthio)-1H-benzimidazoyl carboxylate of formula VIII

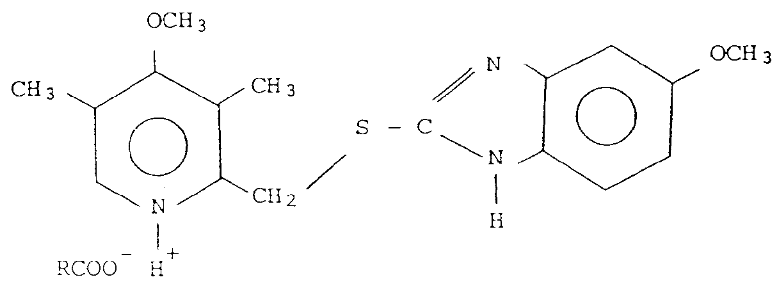

(VIII)

where RCOOH is an alkylcarboxylic acid, with the oxidation of the formula VIII compound leading to omeprazol of formula I.

**11.-** The process of any one of claims 1 to 9, comprising successively the following steps: a) allyl chlorination of the 2-position methyl group, in the compound of formula III by reaction with a halogenating agent, to obtain a compound of formula IX

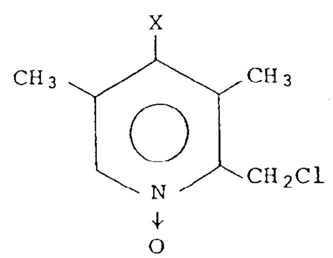

(IX)

where X is a halogen; b) nucleophilic substitution of the -Cl radical of the 2-position methyl group by the -SR radical, where R has the meaning given above, by reaction of the compound of formula IV with 5-methoxy-2-mercapto-benzimidazole of formula V to give 5-methoxy-2-((3,5-dimethyl-4-halo-2-pyridinyl-N-oxide)methylthio)-1H-benzimidazole of formula X

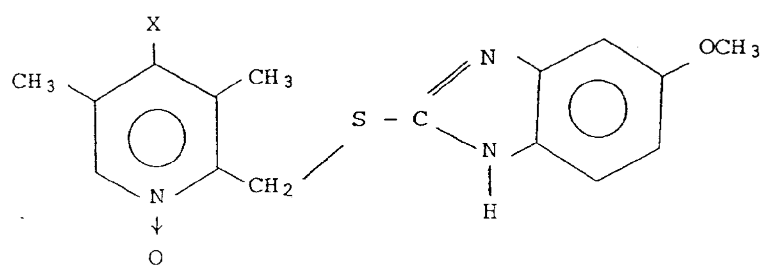

(X)

c) deoxygenation of the compound of formula X by reaction with a reducing compound to give 5-methoxy-2-((3,5-dimethyl-4-halo-2-pyridinyl)methylthio)-1H-benzimidazole of formula XI

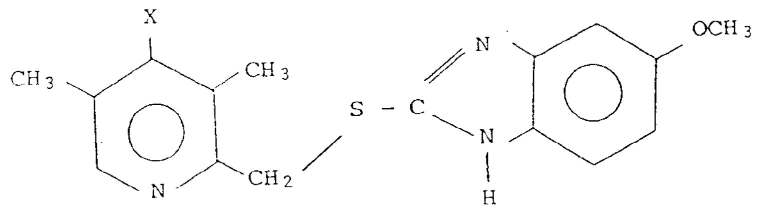

(XI)

and d) nucleophilic substitution of the halogen group by the radical $-OCH_3$, which when treated with a carboxylic acid gives the corresponding 5-methoxy-2-((3,5-dimethyl-4-methoxy-2-pyridinyl)methylthio)-1H-benzimidazoyl carboxylate of formula VIII, oxidation of which leads to omeprazol of formula I.

**12.-** The process of any one of claims 1 to 9, comprising successively the following steps: a) allyl chlorination of the 2-position methyl group, after neutralization of said salt of a nitrated compound by treatment with a halogenating agent, to give a compound of formula IV; b) nucleophilic substitution of the -Cl radical of the 2-position methyl group by the -SR radical, where R has the meaning given above, by reaction of the compound of formula IV with 5-methoxy-2-mercapto-benzimidazole of formula V to give 5-methoxy-2-((3,5-dimethyl-4-nitro-2-pyridinyl-N-oxide)methylthio)-1H-benzimidazole of formula VI; c) nucleophilic substitution of the $NO_2$ group by the radical $-OCH_3$, which leads to 5-methoxy-2-((3,5-dimethyl-4-methoxy-2-pyridinyl-N-oxide)methylthio)-1H-benzimidazole of formula XII

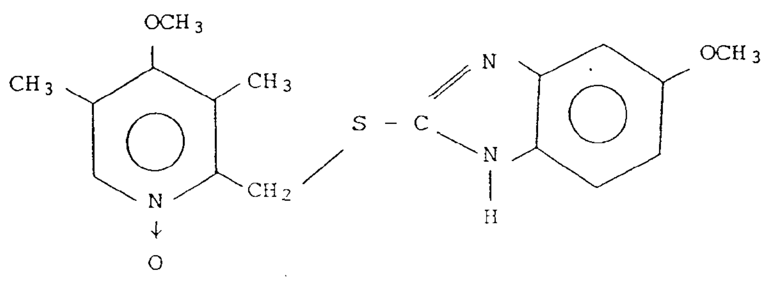

(XII)

and d) deoxygenation of the compound of formula XII, by reaction with a reducing compound, which when treated with a carboxylic acid gives the corresponding 5-methoxy-2-((3,5-dimethyl-4-nitro-2-pyridinyl)methylthio)-1H-benzimidazoyl carboxylate of formula VIII, oxidation of which leads to omeprazol of formula I.

**13.-** The process of any one of claims 1 to 9, comprising successively the following steps: a) allyl chlorination of the 2-position methyl group, of a compound of formula III by reaction with a halogenating agent, to give a compound of formula IX; b) nucleophilic substitution of the -Cl radical of the 2-position methyl group, by the -SR radical, where R has the meaning given above, by reaction of the compound of formula IX with 5-methoxy-2-mercapto-benzimidazole of formula V to give 5-methoxy-2-((3,5-dimethyl-4-halo-2-pyridinyl-N-oxide)methylthio)-1H-benzimidazole of formula X; c) nucleophilic substitution of the halogen by the radical $-OCH_3$, which leads to 5-methoxy-2-((3,5-dimethyl-4-methoxy-2-pyridinyl-N-oxide) methylthio)-1H-benzimidazole of formula XII and d) deoxygenation of the compound of formula XII, by reaction with a reducing compound, which when treated with a carboxylic acid gives the corresponding 5-methoxy-2-((3,5-dimethyl-4-nitro-2-pyridinyl)methylthio)-1H-benzimidazoyl carboxylate of formula VIII, oxidation of which leads to omeprazol of formula I.

**14.-** The process of any one of claims 1 to 9, comprising successively the following steps: a) allyl chlorination of the 2-position methyl group, after neutralization of said salt of a nitrated compound by reaction with a halogenating agent, to give a compound of formula IV; b) deoxygenation of the compound of formula IV, by reaction with a reducing compound to give 2-chloromethyl-3,5-dimethyl-4-nitro-pyridine hydrochloride of formula XIII

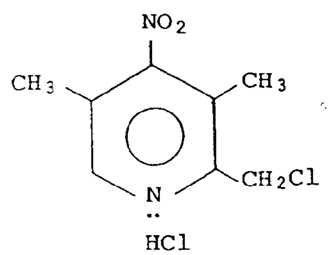

(XIII)

c) nucleophilic substitution of the -Cl radical of the 2-position methyl group, with the -SR radical, where R has the meaning given above, by reaction of the compound of formula XIII with 5-methoxy-2-mercapto-benzimidazole of formula V, to give the compound of formula VII; and d) nucleophilic substitution of the $NO_2$ group by the $-OCH_3$ radical, which by treatment with a carboxylic acid gives the corresponding 5-methoxy-2-((3,5-dimethyl-4-methoxy-2-pyridinyl)methylthio)-1H-benzimidazoyl carboxylate of formula VIII, oxidation of which gives the omeprazol of formula 1.

**15.-** The process of any one of claims 1 to 9, comprising successively the following steps: a) allyl chlorination of the 2-position methyl group, of a compound of formula III by reaction with a halogenating agent, to give the compound of formula IX; where X is a halogen; b) deoxygenation of the compound of formula IX by reaction with a reducing compound, to give the 2-chloromethyl-3,5-dimethyl-4-halo-pyridine hydrochloride of formula XV

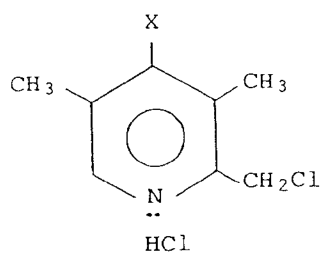

(XV)

c) nucleophilic substitution of the -Cl radical of the 2-position methyl group, by the -SR radical, where R has the meaning given above, by reaction of the compound of formula XV with 5-methoxy-2-mercapto-benzimidazole of formula V, to give the compound of formula XI; and d) nucleophilic substitution of the halogen group by the $-OCH_3$ radical, which by treatment with the carboxylic acid gives the corresponding 5-methoxy-2-((3,5-dimethyl-4-methoxy-2-pyridinyl)methylthio)-1H-benzimidazoyl of formula VIII, oxidation of which gives the omeprazol of formula I.

**16.-** The process of any one of claims 1 to 9, comprising successively the following steps: a) deoxygenation, after neutralization, of said salt of a nitrated compound by reaction with a reducing compound to give 2,3,5-trimethyl-4-nitro-pyridine of formula XVII

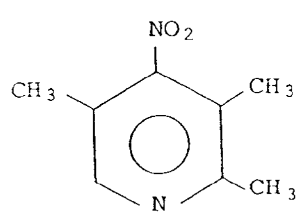

(XVII)

b) allyl chlorination of the 2-position methyl group, of the compound of formula XVII, by reaction with a halogenating agent to give 2-chloromethyl-3,5-dimethyl-4-nitro-pyridine of formula XVIII

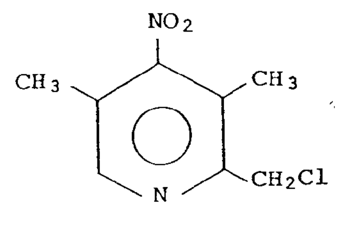

(XVIII)

c) nucleophilic substitution of the -Cl radical by the -SR radical, where R has the meaning given above, by reaction of the compound of formula XVIII with 5-methoxy-2-mercapto-benzimidazole of formula V, to give 5-methoxy-2-((3,5-dimethyl-4-nitro-2-pyridinyl)methylthio-1H-benzimidazole of formula VII; and d) nucleophilic substitution of the $NO_2$ group by the radical $-OCH_3$ , which by treatment with a carboxylic acid gives the corresponding 5-methoxy-2-((3,5-dimethyl-4-methoxy-2-pyridinyl)methylthio)-1H-benzimidazoyl carboxylate of formula VIII, oxidation of which gives the omeprazol of formula I.

**17.-** The process of any one of claims 1 to 9, comprising successively the following steps: a) deoxygenation of a compound of formula III by reaction with a reducing compound, to give 2,3,5-trimethyl-4-halo-pyridine of formula XIX

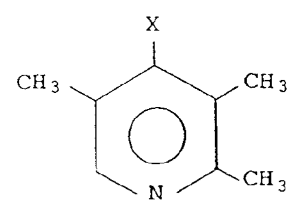

(XIX)

b) allyl chlorination of the 2-position methyl group, of the compound of formula XIX by reaction with a halogenating agent, to give 2-chloromethyl-3,5-dimethyl-4-nitro-pyridine of formula XX

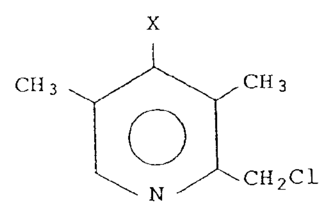

(XX)

c) nucleophilic substitution of the -Cl radical of the 2-position methyl group, by the -SR radical, where R has the meaning given above, by reaction of the compound of formula XX with 5-methoxy-2-mercapto-benzimidazole of formula V, to give 5-methoxy-2-((3,5-dimethyl-4-halo-2-pyridinyl)methylthio-1H-benzimidazole of formula XI; and d) nucleophilic substitution of the halogen group by the $-OCH_3$ radical, which by treatment with a carboxylic acid gives the corresponding 5-methoxy-2-((3,5-dimethyl-4-methoxy-2-pyridinyl)methylthio)-1H-benzimidazoyl carboxylate of formula VIII, oxidation of which gives the omeprazol of formula I.

**18.-** The process of any one of claims 1 to 9, comprising successively the following steps: a) nucleophilic substitution of the $NO_2$ group by the $-OCH_3$ radical in said salt of a nitrated compound by reaction with sodium methoxide, to give an intermediate which is not isolated; b) allyl chlorination of the 2-position methyl group, after neutralization of said intermediate, by a halogenating agent to give the 2-chloromethyl-3,5-dimethyl-4-nitro-pyridine N-oxide of formula XXII

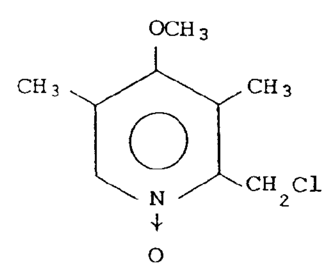

(XXII)

c) nucleophilic substitution of the -Cl radical by the -SR radical where R has the meaning given above by reaction of the compound of formula XXII with 5-methoxy-2-mercapto-benzimidazole of formula V to give the compound of formula XXIII

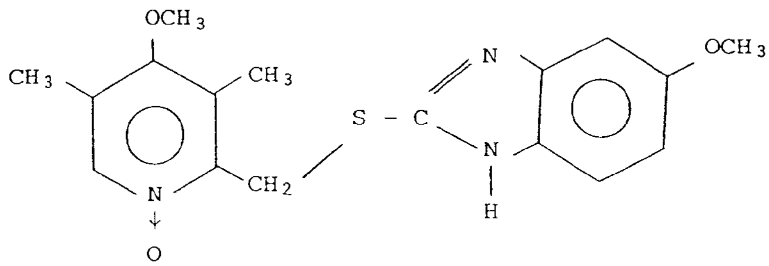

(XXIII)

d) deoxygenation of the compound of formula XXIII by reaction with a reducing compound, which by treatment with a carboxylic acid gives the compound of formula VIII, oxidation of which leads to omeprazol of formula I.

**19.-** The process of any one of claims 1 to 9, comprising successively the following steps: a) nucleophilic substitution of the halogen group by the $-OCH_3$ radical, in a compound of formula III by reaction with sodium methoxide, to give an intermediate which is not isolated; b) allyl chlorination of the 2-position methyl group, of said intermediate, by a halogenating agent to give 2-chloromethyl-3,5-dimethyl-4-methoxypyridine N-oxide of formula XXII

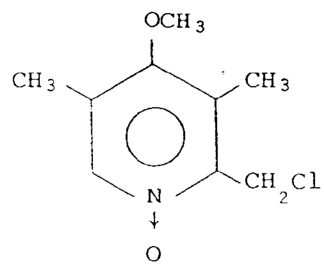

(XXII)

c) nucleophilic substitution of the -Cl radical by the -SR radical, where R has the meaning given above; by reaction of the compound of formula XXII with 5-methoxy-2-mercapto-benzimidazole of formula V to give the compound of formula XXIII

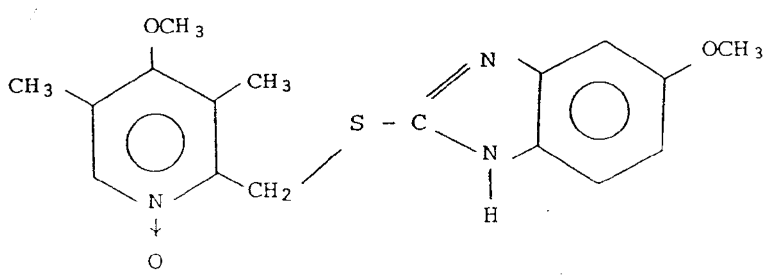

(XXIII)

d) deoxygenation of the compound of formula XXIII by reaction with a reducing compound, which after treatment with a carboxylic acid gives the compound of formula VIII, oxidation of which gives the omeprazol of formula I.

**20.-** The process of any one of claims 1 to 9, comprising successively the following steps: a) nucleophilic substitution of the $NO_2$ group by the $-OCH_3$ radical, in said salt of a nitrated compound by reaction with sodium methoxide, to give an intermediate which is not isolated; b) allyl chlorination of the 2-position methyl group, of said intermediate, with a halogenating agent, to give 2-chloromethyl-3,5-dimethyl-4-methoxypyridine N-oxide of formula XXII

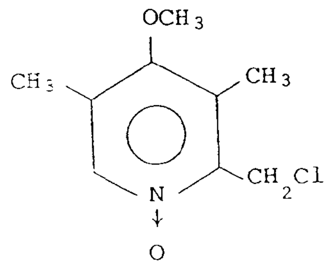

(XXII)

c) deoxygenation of the compound of formula XXII by reaction with a reducing compound to give 2-chloromethyl-3,5-dimethyl-4-methoxy-pyridine hydrochloride of formula XXIV

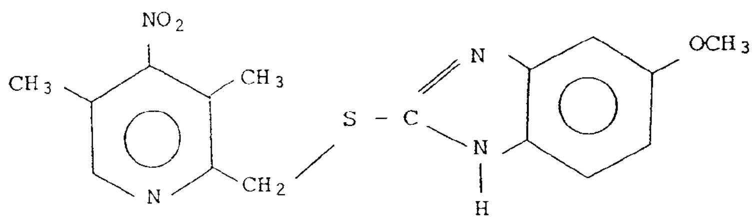

(XIV)

d) nucleophilic substitution of the -Cl radical of the 2-position methyl group, by the -SR radical, where R has the meaning given above, by reaction of the compound of formula XXIV with 5-methoxy-2-mercapto-benzimidazole of formula V, which by treatment with a carboxylic acid gives the compound of formula VIII, oxidation of which gives the omeprazol of formula I.

**21.-** The process of any one of claims 1 to 9, comprising successively the following steps: a) nucleophilic substitution of the halogen group by the $-OCH_3$ radical in a compound of formula III by reaction with sodium methoxide to give an intermediate which is not isolated; b) allyl chlorination of the 2-position methyl group, of said intermediate, with a halogenating agent, to give 2-chloromethyl-3,5-dimethyl-4-nitropyridine N-oxide of formula XXII; c) deoxygenation of the compound of formula XXII by reaction with a reducing compound to give 2-chloromethyl-3,5-dimethyl-4-methoxypyridine hydrochloride of formula XXIV; d) nucleophilic substitution of the -Cl radical of the 2-position methyl group, by the -SR radical, where R has the meaning given above, by reaction of the compound of formula XXIV with 5-methoxy-2-mercapto-benzimidazole of formula V, which by treatment with a carboxylic acid gives the compound of formula VIII, oxidation of which gives the omeprazol of formula I.

**22.-** The process of any one of claims 1 to 9, comprising successively the following steps: a) O-acylation and subsequent acetoxylation of said salt of a nitrated compound, by means of acetic acid and acetic anhydride to give 2-acetoxymethyl-3,5-dimethyl-4-nitropyridine of formula XXV

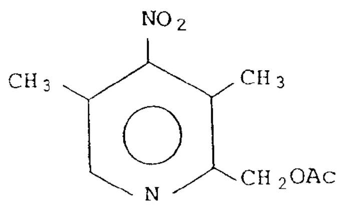

(XXV)

b) acid hydrolysis of the compound of formula XXV to give 2-hydroxymethyl-3,5-dimethyl-4-nitropyridine hydrochloride of formula XXVI

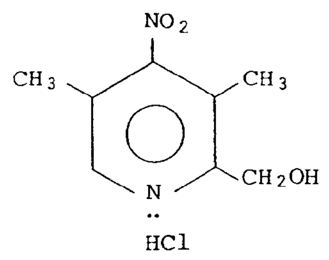

(XXVI)

c) nucleophilic substitution of the -OH radical by the -Cl radical by treatment with $SOCl_2$, to give 2-chloromethyl-3,5-dimethyl-4-chloropyridine hydrochloride of formula XXVII

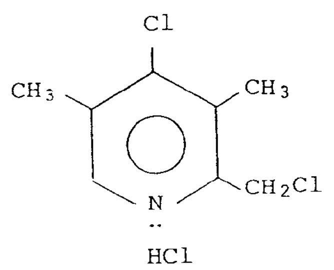

(XXVII)

d) nucleophilic substitution of the -Cl radical of the 2-position methyl group, by the -SR radical, where R has the meaning given above, by reaction of the compound of formula XXVII with 5-methoxy-2-mercapto-benzimidazole of formula V, to give the compound of formula XXVIII

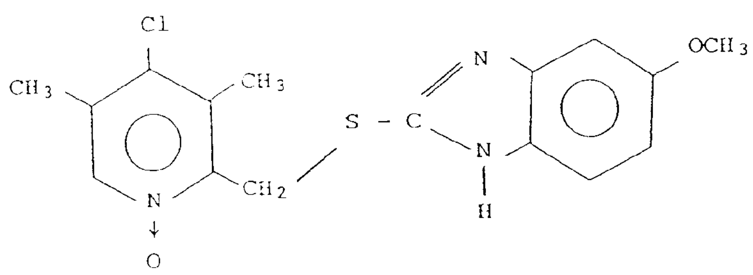

(XXVIII)

and e) nucleophilic substitution of the -Cl group by the $-OCH_3$ radical by reaction of the compound of formula X with sodium methoxide which, by treatment with a carboxylic acid, gives the compound of formula VIII, oxidation of which gives the omeprazol of formula I.

**23.-** The process of any one of claims 1 to 9, comprising successively the following steps: a) O-acylation and subsequent acetoxylation of a compound of formula III with acetic acid and acetic anhydride to give 2-acetoxymethyl-3,5-dimethyl-4-halopyridine of formula XXIX

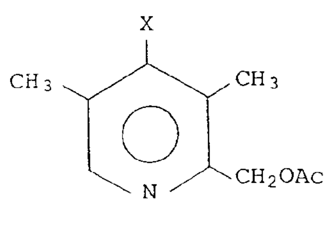

(XXIX)

b) acid hydrolysis of the compound of formula XXIX, giving 2-hydroxymethyl-3,5-dimethyl-4-halopyridine hydrochloride of formula XXX

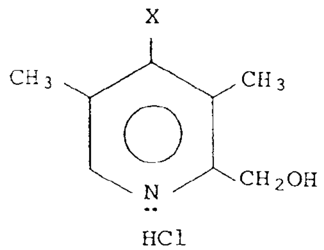

(XXX)

c) nucleophilic substitution of the -OH radical by the -Cl radical by treatment with a $SOCl_2$ to give 2-chloromethyl-3,5-dimethyl-4-halopyridine hydrochloride of formula XV; d) nucleophilic substitution of the -Cl radical of the 2-position methyl group, by the -SR radical, where R has the meaning given above, by reaction of the compound of formula XV with 5-methoxy-2-mercapto-benzimidazole of formula V, to give the compound of formula X; and e) nucleophilic substitution of the halogen group by the $-OCH_3$ radical by the reaction of the compound of formula X with sodium methoxide which, by treatment with carboxylic acid, gives the compound of formula VIII, oxidation of which gives the omeprazol of formula I.

**24.-** The process of any one of claims 10, 12, 14 or 16, wherein said step a) neutralization is carried out by addition of an organic solvent over said salt of a nitrated compound and subsequent treatment with an aqueous alkali solution.

**25.-** The process of any one of claims 10 to 15, wherein said halogenating agent is sulfuryl chloride, in the presence of a catalyst, or an N-chlorinated derivative of an amide or imide.

**26.-** The process of claim 25, wherein said catalyst is Pd/tetraphenylphosphine or benzoyl peroxide.

**27.-** The process of claim 16 or 17, wherein said halogenating agent is an N-chlorinated derivative of an amide or imide.

**28.-** The process of any one of claims 25 or 27, wherein said N-chlorinated derivative is N-chlorosuccinimide, N-chlorobenzamide or trichloroisocyanuric acid.

**29.-** The process of any one of claims 18 to 21, wherein said halogenating agent is N-chlorosuccinimide.

**30.-** The process of any one of claims 25 to 29, wherein the halogenating reaction is carried out in an organic solvent at a temperature ranging from 0°C to the reflux temperature of the solvent.

**31.-** The process of any one of claims 10 to 23, wherein the reaction with 5-methoxy-2-mercapto-benzimidazole of formula V is carried out in an organic solvent, preferably methanol, dimethylsulfoxide, sulfolane, dimethyl- acetamide or mixtures thereof

**32.-** The process of claim 31, wherein said reaction is carried out in the presence of sodium methoxide and optionally in presence of catalytic amounts of quaternary ammoniums, crown ethers or quaternary phosphoniums.

**33.-** The process of any one of claims 10 to 21, wherein said reducing agent is phosphorous trichloride, phosphorous tribromide, sulfur dichloride or sulfurous anhydride.

**34.-** The process of claim 33, wherein the amounts of reducing agent used are stoichiometric or in a slight excess, and the reaction temperatures range from -40°C to room temperature.

**35.-** The process of any one of claims 10 to 17 or 23, wherein the nucleophilic substitution of the pyridine ring 4-position substituent is carried out by addition of sodium methoxide in the presence of a solvent and of a catalyst.

**36.-** The process of any one of claims 10, 12, 14, 16 or 18 to 22, wherein the nucleophilic substitution of the pyridine ring 4-position substituent is carried out by addition of sodium methoxide in the presence of methanol and of a catalyst.

**37.-** The process of claim 35, wherein said solvent is dimethylsulfoxide, sulfolane, N,N-dimethylacetamide or mixtures thereof or with methanol.

**38.-** The process of any one of claims 35 or 36, wherein said catalyst is a quaternary ammonium salt, a quaternary phosphonium salt or a crown ether, such that the nucleophilic reactant of said substitution is the free anion $CH_3O^-$.

**39.-** The process of any one of claims 22 or 23, wherein the O-acylation and subsequent acetoxylation is carried out by addition of acetic acid and acetic anhydride in the presence of a catalyst at a temperature ranging from 80 to 120 °C.

**40.-** The process of claim 39, wherein said catalyst is a 4-dialkylaminopyridine, such as 4-dimethylaminopyridine, 4-pyrrolidinepyridine or 4-guanidinepyridine.

**41.-** The process of any one of claims 22 or 23, wherein said acid hydrolysis is carried out with an aqueous inorganic acid, preferably hydrochloric acid.

**42.-** The process of any one of claims 22 or 23, wherein said nucleophilic substitution of the -OH radical by the -Cl radical is carried out by addition of thionyl chloride in the presence of methylene chloride as solvent and of a catalyst.

**43.-** The process of claim 42, wherein said catalyst is an amide, preferably dimethylformamide.

**44.-** The process of any one of claims 10 to 23, wherein said alkylcarboxylic acid of which the compound of formula VIII is a salt, is formic, acetic, monochloroacetic, dichloroacetic, pivalic, propanoic or 2-ethylhexanoic acid.

**45.-** The process of claim 44, wherein said formula VIII salt is formed in situ in the solution of the substrate in a solvent, in which the salt is at least partly soluble.

**46.-** The process of claim 45, wherein said solvent is ethyl, methyl or isopropyl acetate, methyl tert.butyl ether, 2,2-dimethoxypropane, acetone or methanol.

**47.-** The process of any one of claims 44 to 46, wherein the oxidation of the compound of formula VIII is carried out at temperatures ranging from -40 to 5°C with a peracid except when the solvent is methanol.

**48.-** The process of claim 47, wherein once the oxidation is completed, the system is stabilized by addition of an organic base, preferably triethylamine.

**49.-** The process of claim 47, wherein said peracid is m-chloroperbenzoic acid, m-nitroperbenzoic acid, m-methoxyperbenzoic acid or magnesium monoperoxyphthalate, which is in the presence of quaternary ammoniums.

**50.-** The process of claim 46, wherein when the solvent is methanol, the oxidation of compound VIII is carried out by addition of an inorganic alkaline base, preferably a carbonate, to basic pH and of $H_2O_2$ in the presence of a catalyst, at a temperature ranging from -10 to 15°C.

**51.-** The process of claim 50, wherein said catalyst is phosphotungstic acid, having formula $H_3(P(W_3O_10)_4).xH_2O$, ammonium molybdate of formula $(NH_4)_2MoO_4$, sodium tungstate of formula $Na_2WO_4$; phosphomolybdic acid of formula $H_3$-$(P(MO_3O_10)_4).xH_2O$; and silicotungstic acid, of formula $H_4(Si(W_3O_10)_4).xH_2O$.

**52.-** The process of any one of claims 47 to 51, wherein the omeprazol of formula I is recrystallized from an organic solvent or mixtures thereof in the presence of an organic base such as triethylamine.

**53.-** The process of claim 52, wherein said solvent is an ester such as ethyl acetate, a ketone such as acetone, an ether such as methyl tert.butyl ether or an alcohol such as methanol.

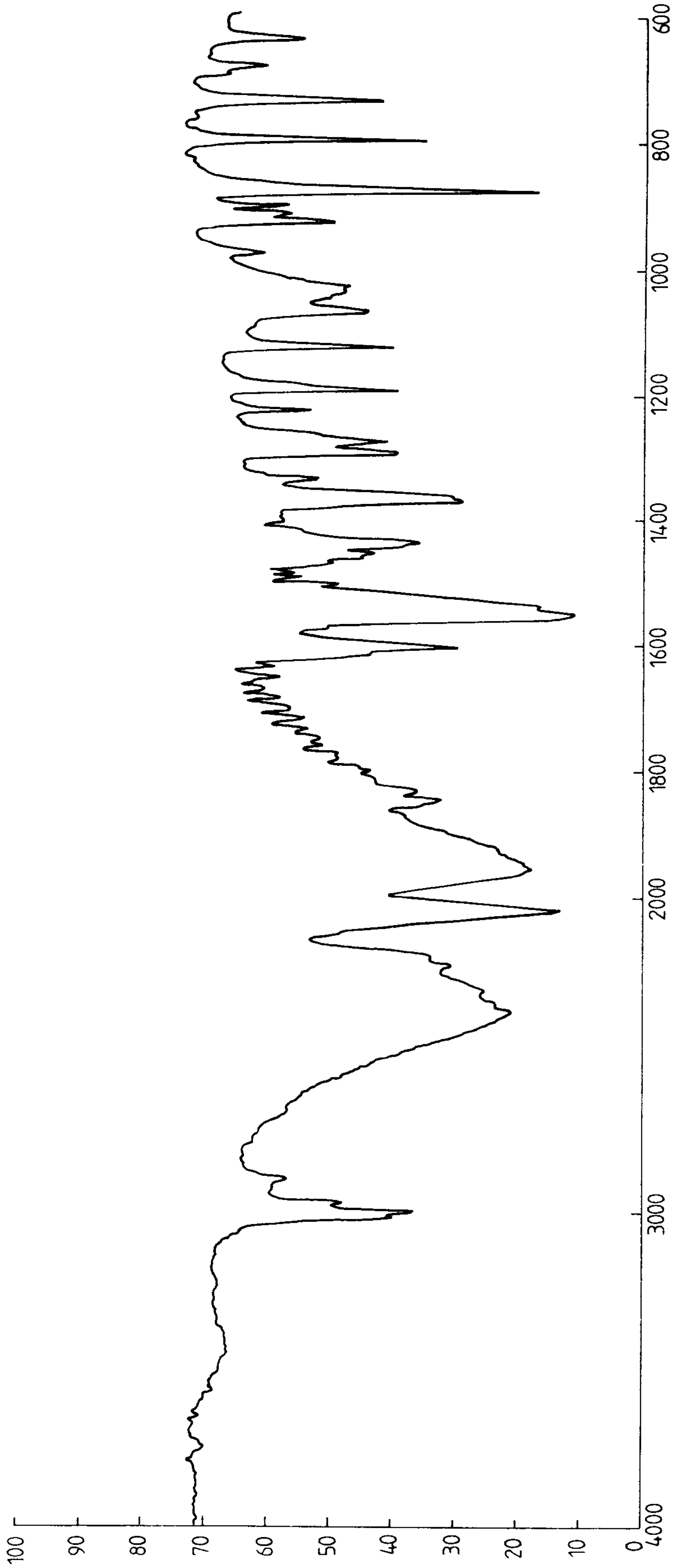
100
90
80
70
60
50
40
30
20
10
0
4000
3000
2000
1800
1600
1400
1200
1000
800
600

FIG. 1

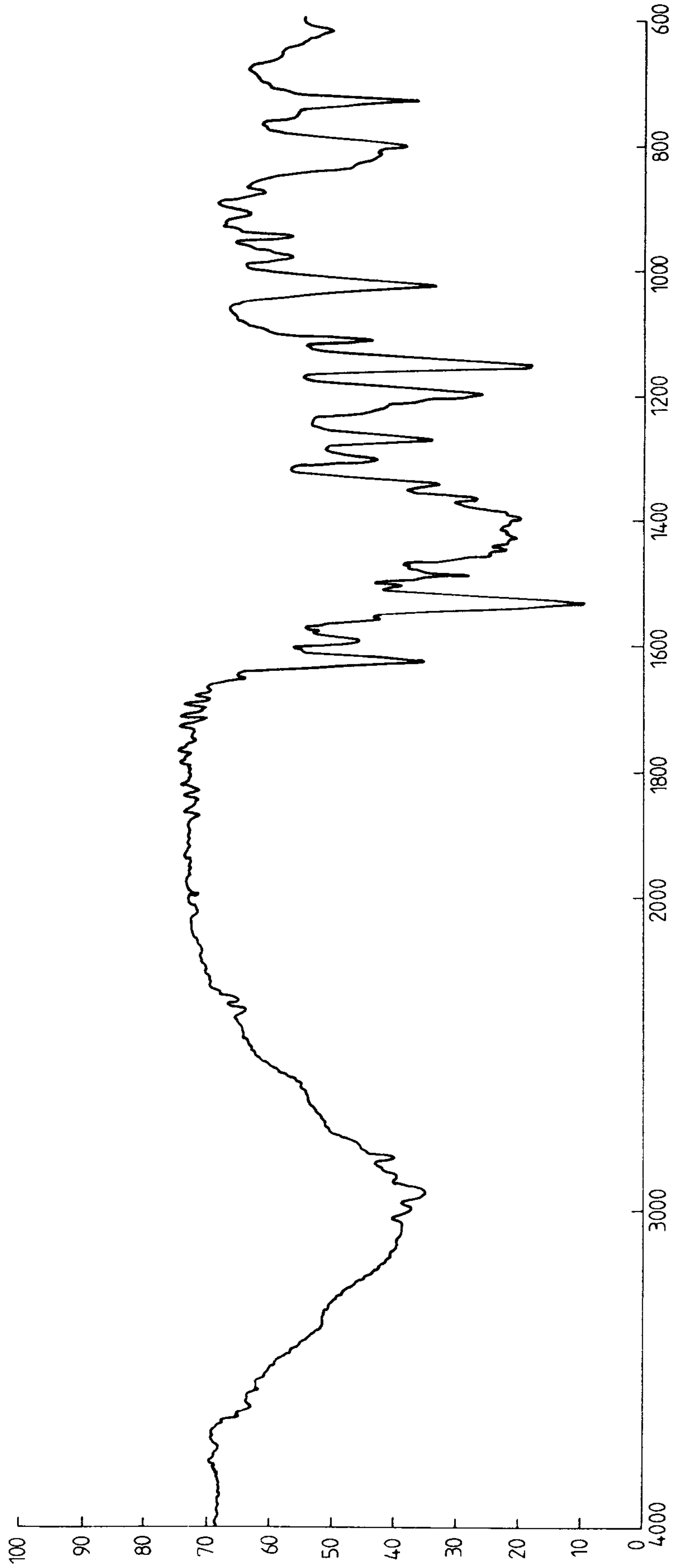
100
90
80
70
60
50
40
30
20
10
0
4000
3000
2000
1800
1600
1400
1200
1000
800
600

FIG. 2

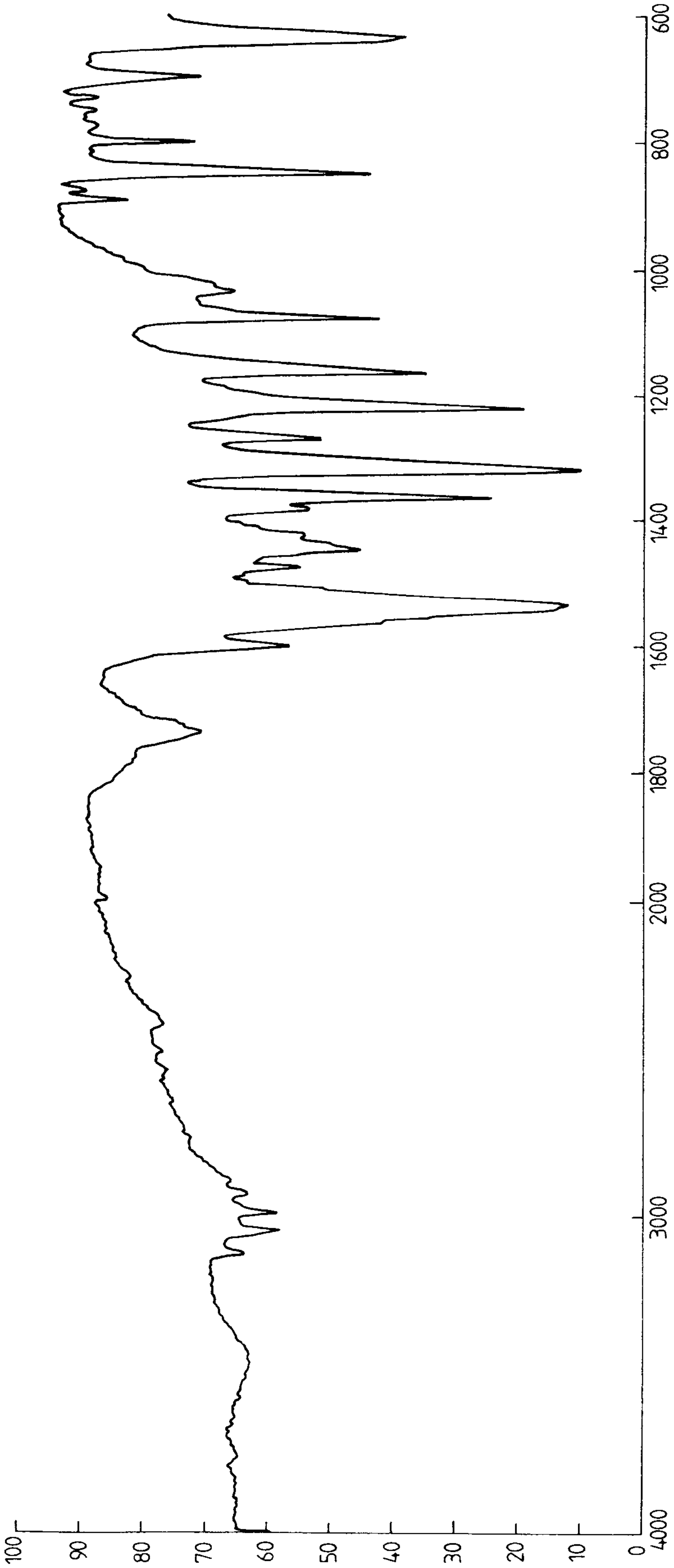
100
90
80
70
60
50
40
30
20
10
0
4000
3000
2000
1800
1600
1400
1200
1000
800
600

FIG. 3

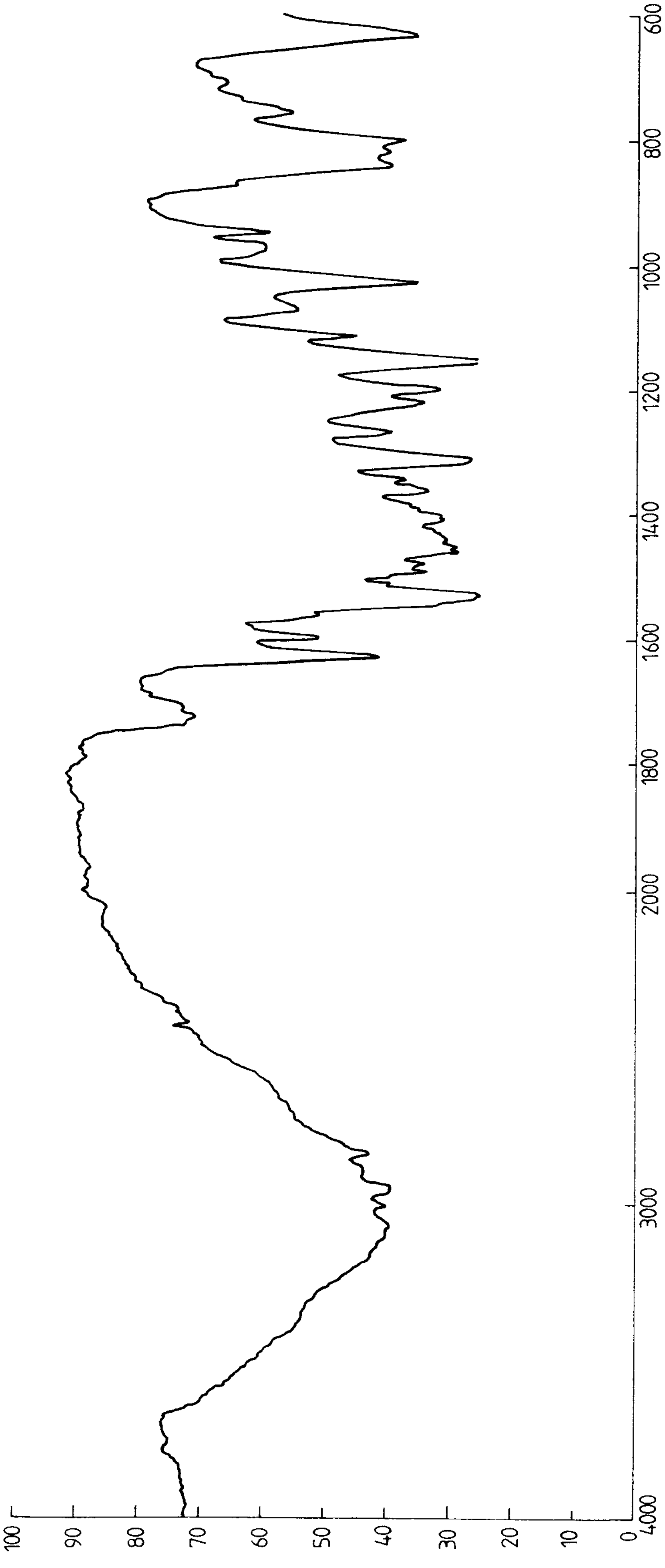
100
90
80
70
60
50
40
30
20
10
0
4000
3000
2000
1800
1600
1400
1200
1000
800
600

FIG. 4

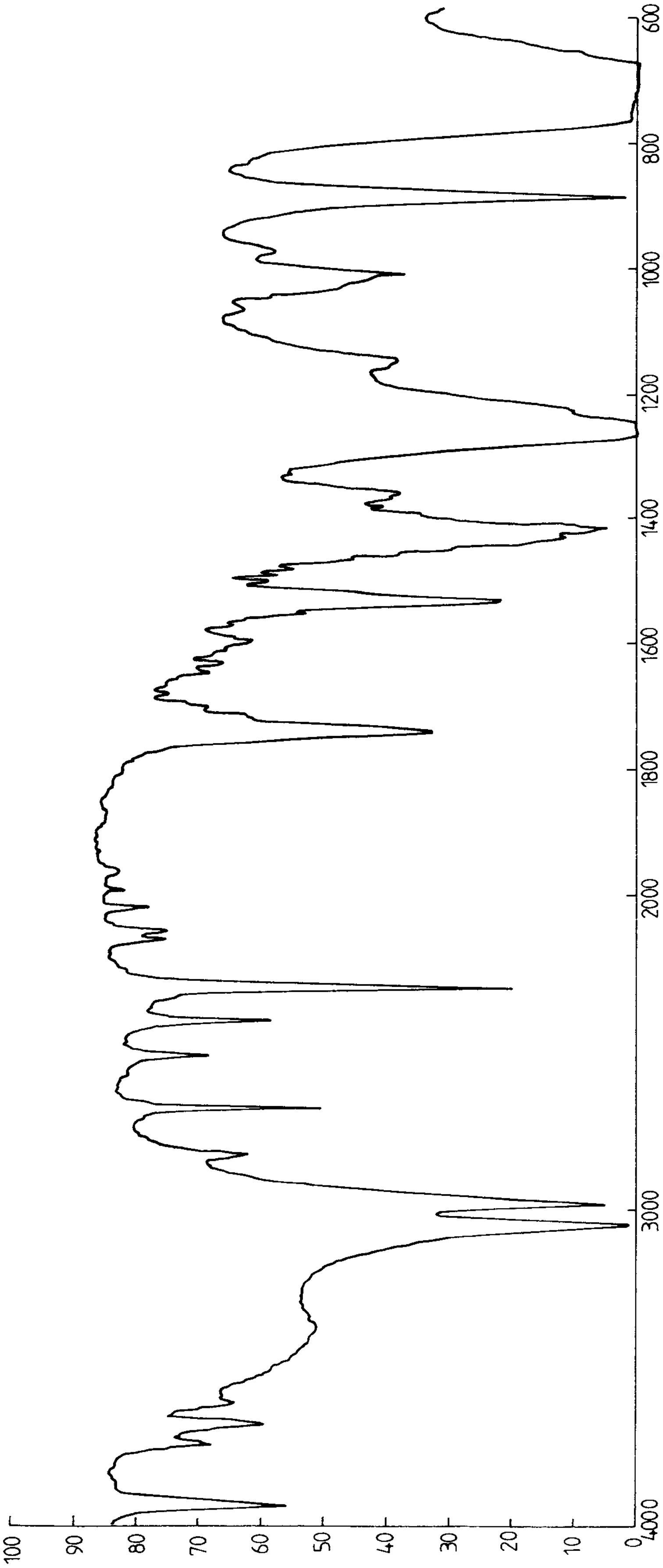
100
90
80
70
60
50
40
30
20
10
0
4000
3000
2000
1800
1600
1400
1200
1000
800
600

FIG. 5

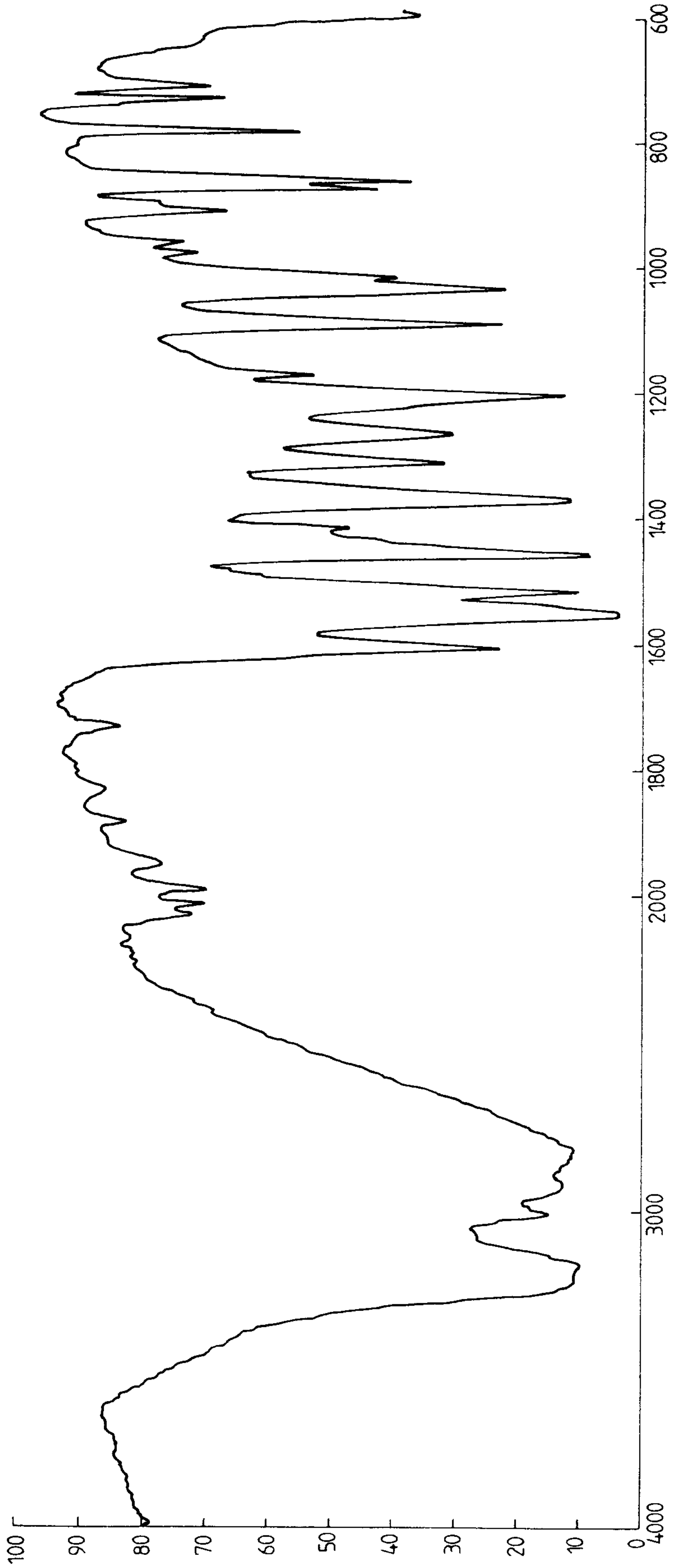
100
90
80
70
60
50
40
30
20
10
0
4000
3000
2000
1800
1600
1400
1200
1000
800
600

FIG. 6

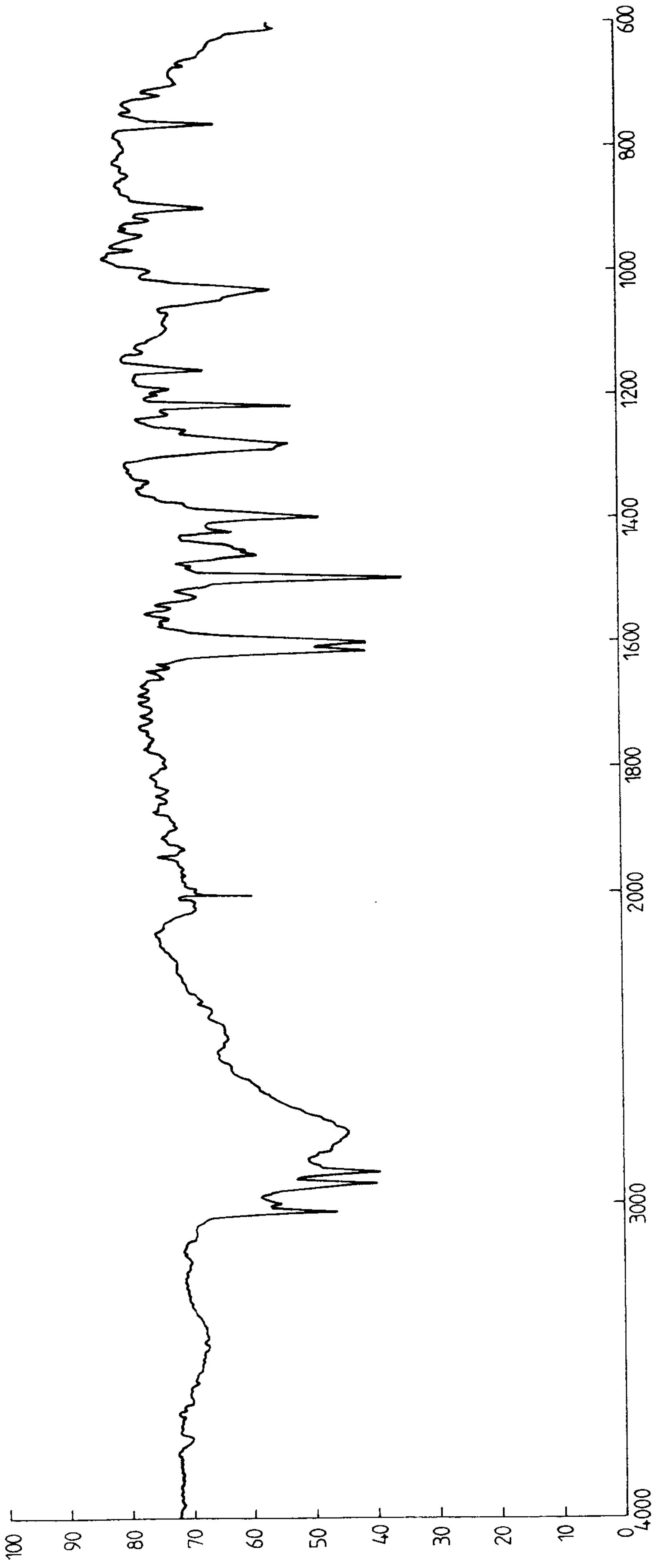
100
90
80
70
60
50
40
30
20
10
0
4000
3000
2000
1800
1600
1400
1200
1000
800
600

FIG. 7

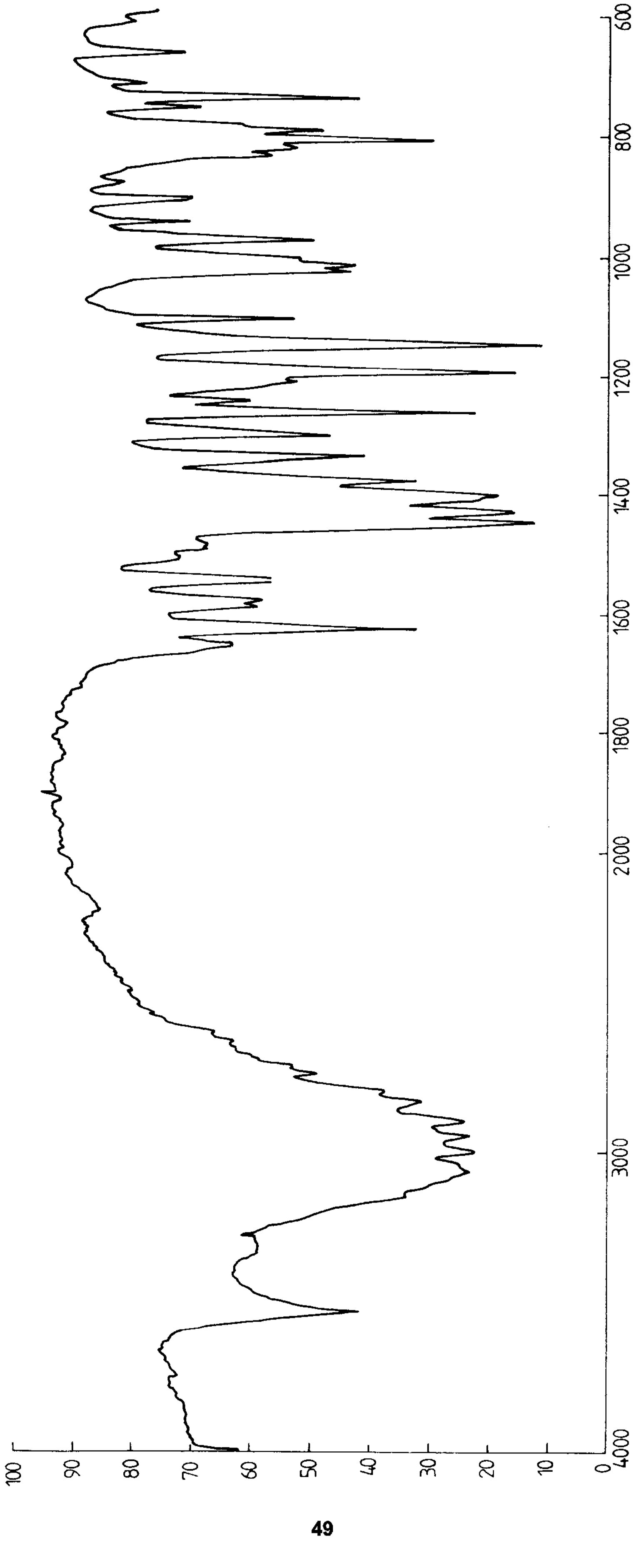
100
90
80
70
60
50
40
30
20
10
0
4000
3000
2000
1800
1600
1400
1200
1000
800
600

FIG. 8

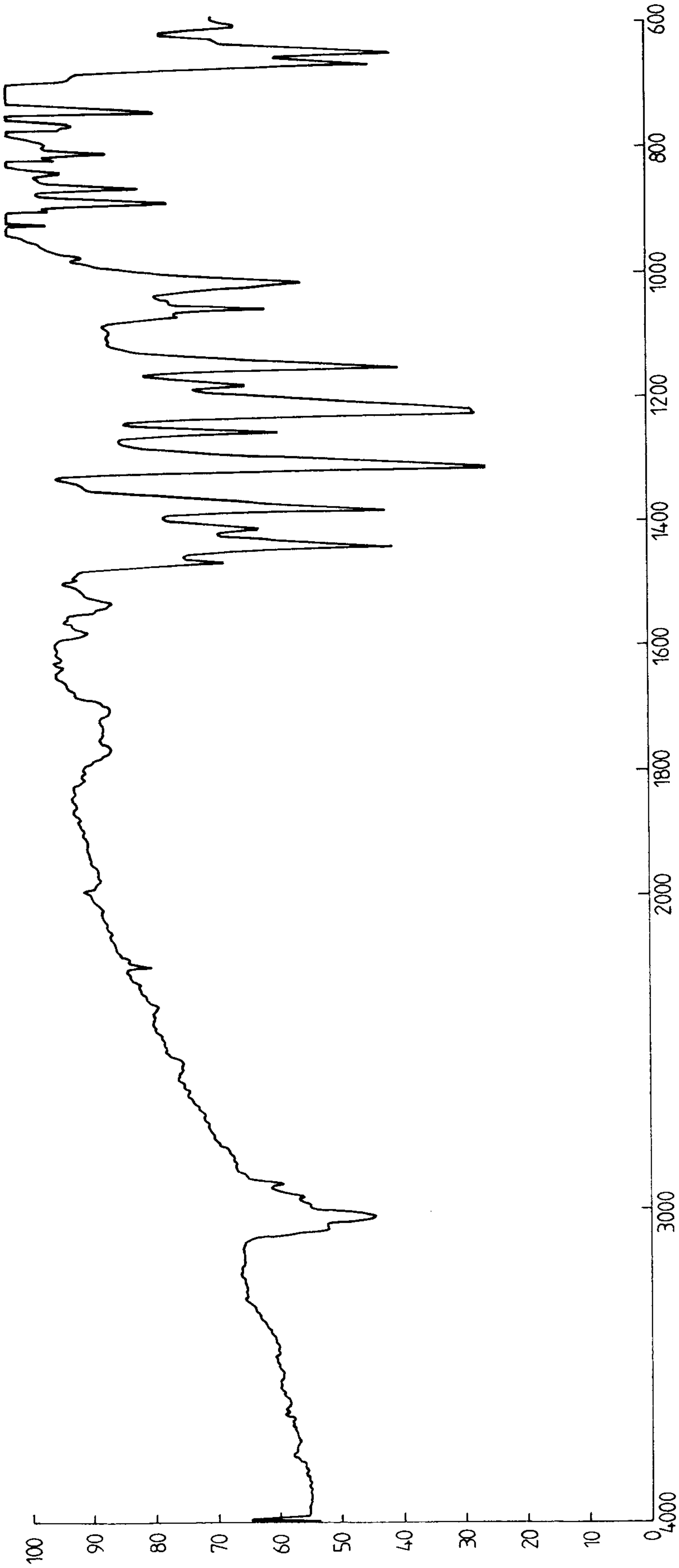

100
90
80
70
60
50
40
30
20
10
0
4000
3000
2000
1800
1600
1400
1200
1000
800
600


FIG. 9

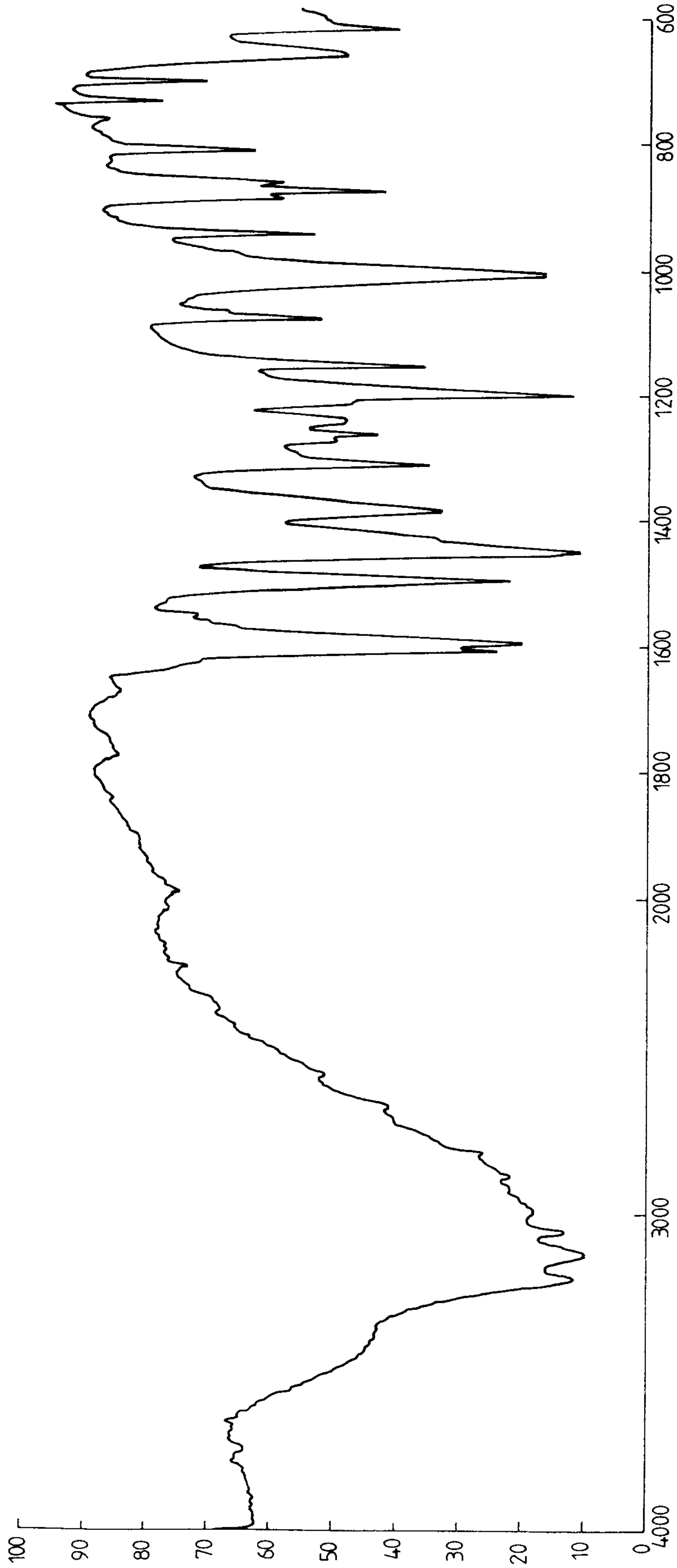
100
90
80
70
60
50
40
30
20
10
0
4000
3000
2000
1800
1600
1400
1200
1000
800
600

FIG. 10

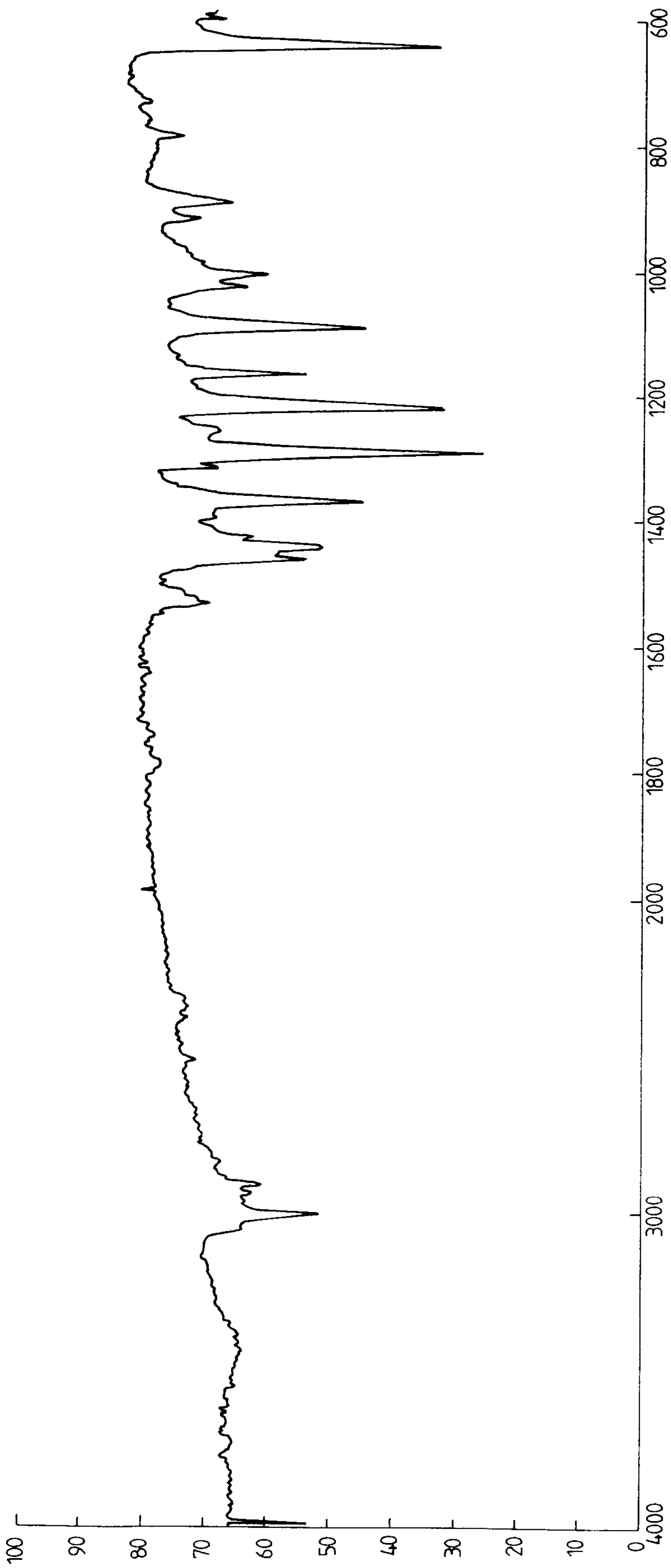
100
90
80
70
60
50
40
30
20
10
0
4000
3000
2000
1800
1600
1400
1200
1000
800
600

FIG. 11

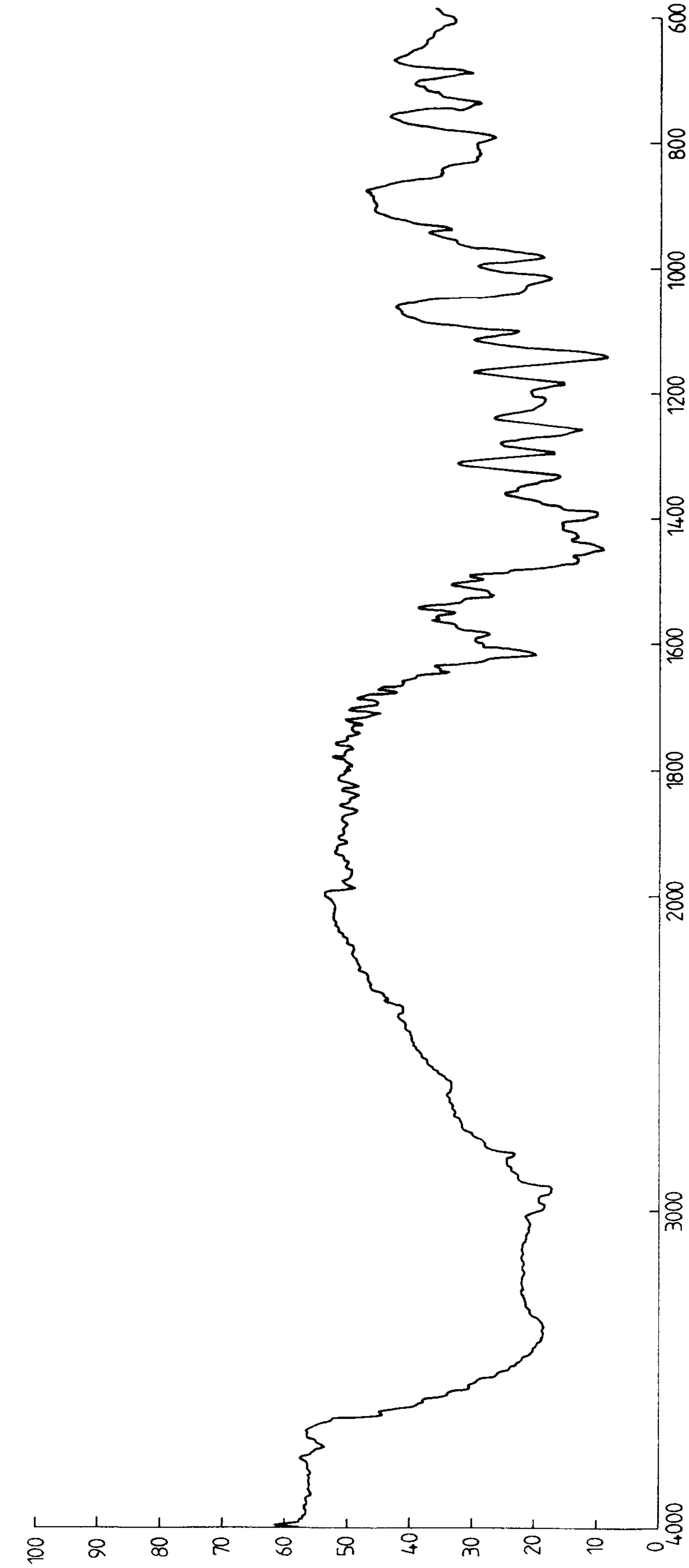
100
90
80
70
60
50
40
30
20
10
0
4000
3000
2000
1800
1600
1400
1200
1000
800
600

FIG. 12

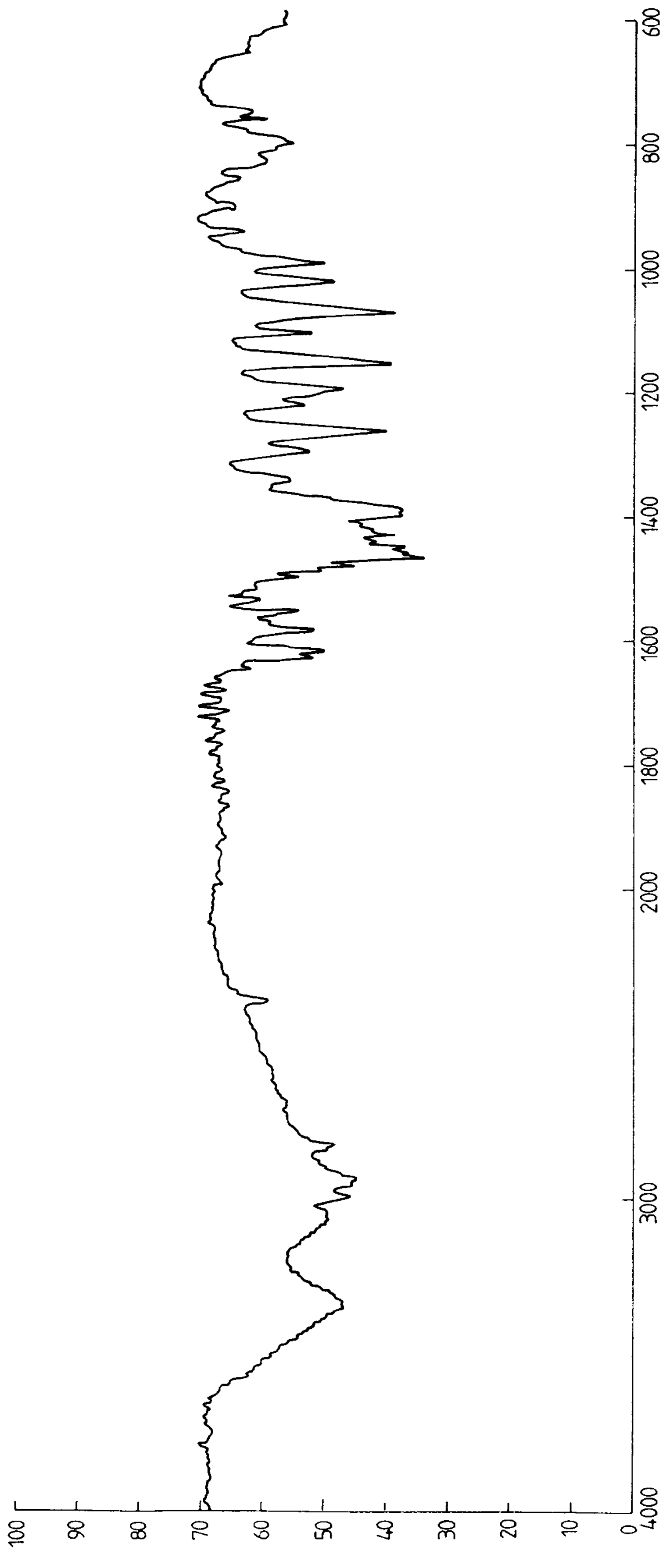
100
90
80
70
60
50
40
30
20
10
0
4000
3000
2000
1800
1600
1400
1200
1000
800
600

FIG. 13

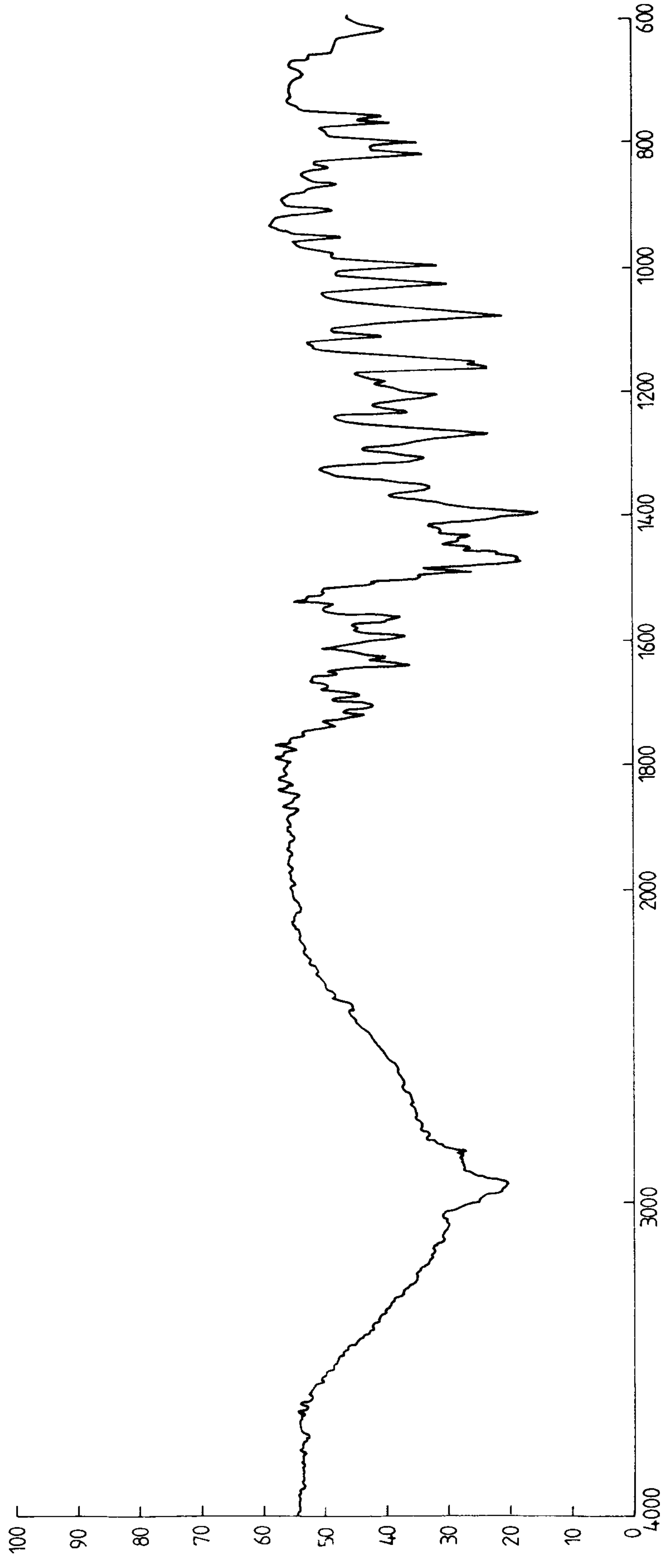
100
90
80
70
60
50
40
30
20
10
0
4000
3000
2000
1800
1600
1400
1200
1000
800
600

FIG. 14

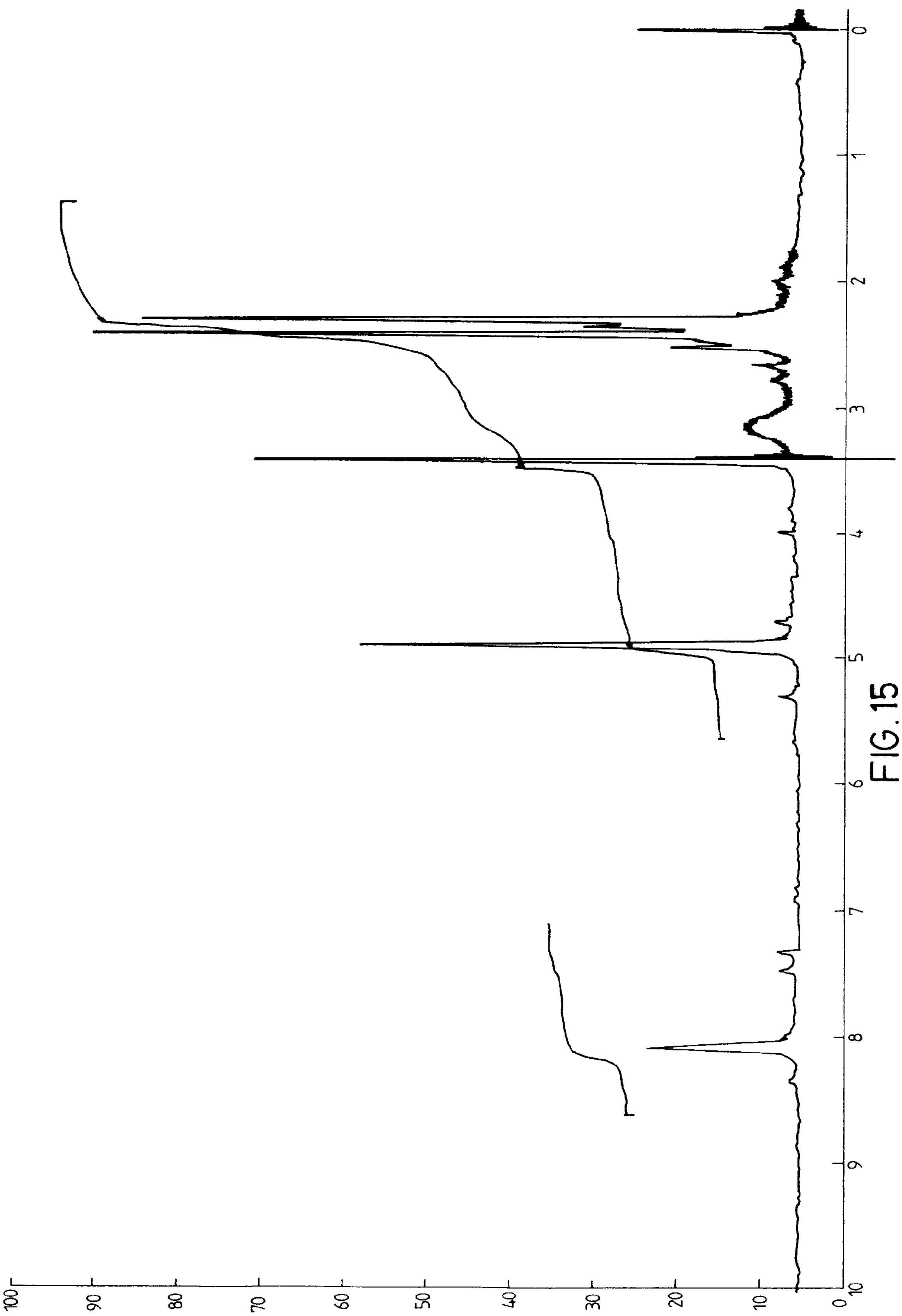


FIG. 15

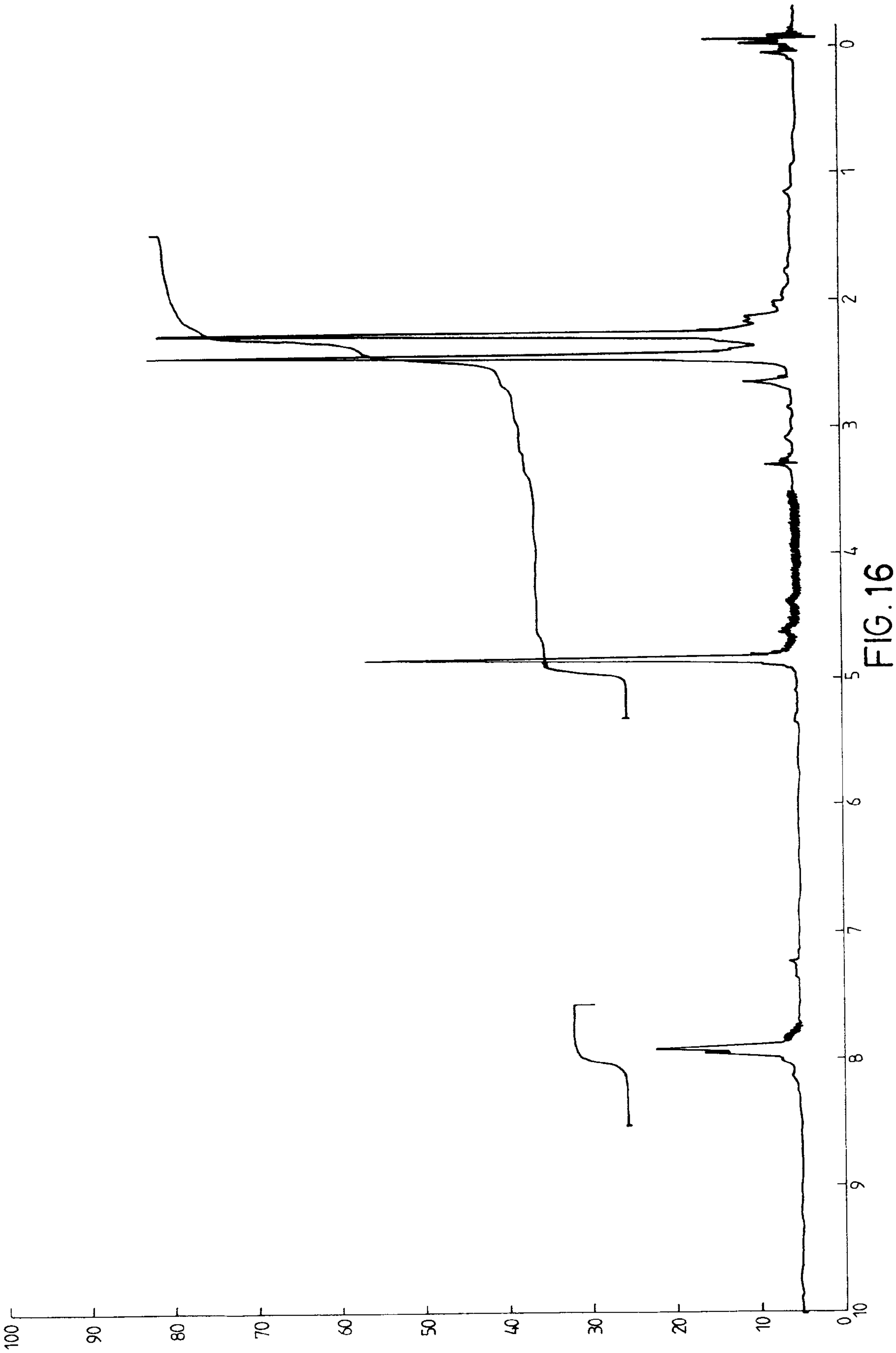


FIG. 16

European Patent Office

# EUROPEAN SEARCH REPORT

Application Number

EP 91 50 0118

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 302 720 (TAKEDA CHEMICAL INDUSTRIES,LTD.)<br>--- | | C07D401/12<br>A61K31/415<br>A61K31/44 |
| A | EP-A-0 124 495 (AKTIEBOLAGET HÄSSLE)<br>--- | | |
| A | WO-A-8 600 913 (BYK GULDEN LOMBERG CHEMISCHE FABRIK GMBH)<br>--- | | |
| A | CHEMICAL ABSTRACTS, vol. 106, no. 1, 5 January 1987, Columbus, Ohio, US; abstract no. 5040E, FERNANDEZ FERNANDEZ M I; IZQUIERDO SANJOSE M; MARTINEZ SANZ A; FUENTES MANSO C; LUCERO DE PABLO M L: 'Benzimidazolyl pyridylmethyl sulfoxide derivatives'<br>page 474 ;column 2 ;<br>* abstract *<br>& ES-A-534 275 (LABORATORIO FARMACEUTICO QUIMICO-LAFAQUIM S.A.) 16 October 1985<br>--- | | |
| D,A | EP-A-0 103 553 (AKTIEBOLAGET HÄSSLE)<br>& ES-A-525 122 (AKTIEBOLAGET HÄSSLE)<br>--- | | TECHNICAL FIELDS SEARCHED (Int. Cl.5 ) |
| D,A | EP-A-0 005 129 (AKTIEBOLAGET HÄSSLE)<br>& ES-A-527 171 (AKTIEBOLAGET HÄSSLE)<br>----- | | C07D<br>A61K |

The present search report has been drawn up for all claims

| Place of search | Date of completion of the search | Examiner |
|---|---|---|
| THE HAGUE | 31 JANUARY 1992 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)